# EUROPEAN PATENT APPLICATION

(11) **EP 4 043 015 A1**
(43) Date of publication of application: **17.08.2022**
(21) Application number: 22165861.0
(22) Date of filing: 04.09.2019
(51) Int. Cl.: A61K 31/355, G01N 33/50, G01N 33/574, A61K 31/616, A61K 45/06, A61K 9/00, A61K 31/713

(54) **DELTA-TOCOTRIENOL FOR TREATING CANCER**

(30) Priority: 04.09.2018 US 201862726665 P
(62) Divisional of application: 19857133.3
(71) Applicant: H. Lee Moffitt Cancer Center And Research Institute, Inc., Tampa, FL 33612-9497 (US)
(72) Inventor: MALAFA, Mokenge, P., Tampa, 33647 (US)
(74) Representative: Dörries, Hans Ulrich

(57) **Abstract**

Disclosed are compositions and methods for inhibiting cancer metastasis or a cancer recurrence in a subject following surgical removal or anti-cancer treatment of a cancer, comprising administering to the subject a composition comprising δ-tocotrienol (d-T3). Also disclosed are methods of determining if a subject is at risk for developing cancer metastasis or recurrence by measuring levels of hCAS expression and an optional treatment step if the subject is identified as being at risk for cancer metastasis or recurrence.

## Description

### CROSS-REFERENCE TO RELATED APPLICATIONS

This application claims the benefit of, and relies on the filing date of, U.S. provisional patent application number 62/726,665, filed 4 September 2018, the entire disclosures of which are incorporated herein by reference.

### BACKGROUND

The treatment of cancer is a highly complicated endeavor, with a significant initial challenge for the patient and treating physician when the first diagnosis is made and followed usually very quickly with a primary treatment regimen. Such regimens can be surgical interventions, radiation treatments, pharmacological interventions, or combinations thereof, depending on the state of the art in the treatment of certain types of cancer at various stages of progression.

If the patient survives the primary intervention, the long-term management of the patient's health must be addressed. Recurrence of the cancer is often a major issue, as the genetic and phenotypic make-up of the patient may carry an inherent risk of developing new cancers as well. For instance, BRCA1/2 mutations are found to be associated with an elevated risk of developing breast or pancreatic cancer. Even if the primary intervention is fully successful, the underlying risk factors may result in the patient developing an entirely new case of cancer over time.

While the primary interventions often reduce the imminent lethality risk for the patient, and in many cases, may provide for a relatively normal life for months-to-years following the primary intervention, many patients remain at risk of a recurrence. Depending on the nature of the cancer or the mode of intervention, the spreading of cancer cells in the form of cancer stem cells (CSC) is a frequent occurrence that typically goes unnoticed until such cells have evolved significantly and establish secondary tumors or spread broadly through various tissues in the body. While the recurrence risk for certain types of cancer that are estrogen modulated may be managed with long term therapy with SERMs (selective estrogen receptor modulators) like tamoxifen and raloxifene, the side effects of these medications can be significant and most cancers or their mutations are not modulated by estrogen. This leaves a significant unmet medical need.

While managing recurrence and prevention of new cancers in high risk individuals are two main areas of high unmet medical need, the treatment of established cancers is often complicated by mutations of the primary cancer under the treatment pressure of chemotherapeutic agents or radiation. These mutations can result in the generation of fresh stem cells of a new nature with a significant degree of resistance to the patient's ongoing treatment regimens. This can greatly complicate treatment options, where the physician is dealing with multiple cancers at the same time, some appearing in the regular cancers screens while others may escape detection during the early stage. Managing the ongoing emergence of mutated forms of the primary cancer into rapidly spreading cancer stem cells is an area of significant unmet need while treating patients diagnosed with cancer. Again, this leaves a significant unmet medical need.

Although many types of cancers (usually described by the organ in which the primary cancer was diagnosed) are unique in nature, biology and physiology, requiring a tailored approach to treatment, some cancers share intracellular mechanisms during the early CSC stage. Targeting these common intracellular mechanisms is a unique mode of action associated with the methods and compositions presented herein.

Colorectal Cancer (CRC) is an important public health problem worldwide. Approximately 1.3 million individuals are newly diagnosed with CRC every year and approximately 614,000 individuals are expected to die of the disease annually. Preventive measures to lower the chances of getting CRC and/or the chances of developing relapse after successful treatment of CRC, even at a modest goal of decreasing the risk of death from CRC by 20%, translate into saving approximately 120,000 lives annually.

Three main strategies have been used to prevent CRC. The most effective strategy, screening individuals with colonoscopy and removing precancerous polyps, has been the main reason for declining incidence rates of CRC in countries where this strategy has been successfully implemented. However, even in advanced countries such as the United States, only about half of eligible individuals undergo a screening colonoscopy. A second strategy, implementing diet and lifestyle changes to minimize the risk of CRC, involves the obvious difficulties in implementing behavior changes in large populations. The third strategy is the use of chemoprevention agents. However, due to severe gastrointestinal (GI) and cardiovascular toxicities, such agents have not been widely adopted for this purpose. Thus, a critical gap remains to develop new and safer strategies for preventing and managing the recurrence and metastatic risks in CRC, as well as other cancers that share the same intracellular mechanisms as CRC, particularly in the CSC stages.

### SUMMARY

δ-tocotrienol (d-T3) has been identified as a safe and effective agent for CRC prevention in preclinical studies. While d-T3 was active against CRC cancer models, the early stage mechanisms in CRC-derived cancer stem cells (CSCs), as disclosed herein, are shared among CSCs of a wide range of cancers, including, but not limited to: lung cancer, ovarian cancer, cervical cancer, breast cancer, prostate cancer, glioblastoma, melanoma, sarcoma, rectal cancer, liver cancer, pancreatic, or colon cancer. In certain embodiments, the cancer comprises CSCs.

Disclosed are methods and compositions related to inhibiting, reducing, and/or preventing metastasis or a cancer recurrence in a subject following surgical removal of cancer or anti-cancer treatment.

In one aspect, disclosed herein are methods of inhibiting, reducing, and/or preventing a cancer (such as, for example, recurrence of lung, colon, rectal, ovarian, pancreatic, and/or breast cancer) in a subject, cancer metastasis (including primary and secondary metastasis) in a subject, and/or cancer recurrence (such as, for example, recurrence of lung, colon, rectal, ovarian, pancreatic, and/or breast cancer) in a subject following surgical removal of a cancer or anti-cancer treatment (such as with an anti-cancer agent) of a cancer comprising administering to the subject a composition comprising a therapeutically effective amount of δ-tocotrienol (d-T3).

The tocotrienols for the aspects disclosed herein have the formula: 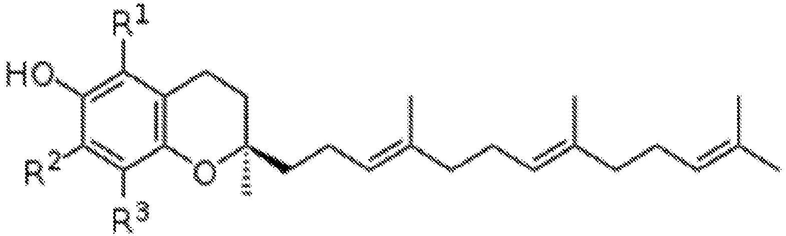 General chemical structure of tocotrienols. *alpha*(*α*)-Tocotrienol: R1 = Me, R2 = Me, R3 = Me; *beta(β)-*Tocotrienol: R1 = Me, R2 = H, R3= Me; *gamma*(*γ*)-Tocotrienol: R1 = H, R2 = Me, R3= Me; *delta*(*δ*)-Tocotrienol: R1 = H, R2 = H, R3= Me

In one aspect, disclosed herein are methods of inhibiting a cancer recurrence of any preceding aspect, further comprising administering to the subject aspirin.

In another aspect, this disclosure provides methods of determining if a subject is at risk for developing cancer metastasis or recurrence, the method comprising assaying a biological sample from the subject to determine a level of hCAS expression in the sample, wherein an increased expression level of hCAS as compared to a control indicates that the subject has an increased risk to develop cancer metastasis or recurrence. The method can further comprises a step of treating the subject.

Also provided are methods and compositions for treating metastasis and cancer recurrence in a subject identified as having increased levels of hCAS. In certain embodiments, the composition comprises a therapeutically effective amount of a compound that reduces expression levels of hCAS, including for example, a nucleic acid inhibitor molecule that targets hCAS and/or δ-tocotrienol (d-T3).

### BRIEF DESCRIPTION OF THE DRAWINGS

The accompanying drawings, which are incorporated in and constitute a part of this specification, illustrate several embodiments and together with the description illustrate the disclosed compositions and methods.
Figure 1A and 1B show the number of intestinal polyps (Fig. 1A) and H&E staining of polyps (Fig. 1B) in ApcMin/+ mice treated with vehicle (V), aspirin (Asp), d-T3, and combination of aspirin + d-T3. Black dots in Fig. 1B point out polyps in the intestine.
Figure 2 shows δ-Tocotrienol administration induced apoptosis in the intestinal stem cells of APCMin/+ mice with or without aspirin administration.
Figures 3A, 3B, 3C and 3D show the number of polyps (Fig. 3A) and cancer (Fig. 3C) with H&E stain of representative areas of polyp (Fig. 3B) and cancer (Fig. 3D) following no treatment (NT), treatment with vehicle (V), sulindac, and d-T3 in AOM-induced Fisher 344 rat model of colon carcinogenesis.
Figure 4A shows the determination of d-T3 IC50 after 5 days treatment in colon cancer stem cells (CCSCs).
Figure 4B shows the pharmacokinetics of d-T3 in humans in a phase I trial.
Figures 5 shows organoids derived from patient's (PI and P2) normal colon, colon tumor, and colon cancer stem cells (CCSCs).
Figures 6A, 6B, 6C, and 6D show a spheroid formation (Fig. 6A) and number of soft agar colony formation (Figs. 6C & 6D) after treatment with vehicle, aspirin, d-T3, and aspirin + d-T3 in CCSCs. Western blot shows expression of stem cell transcription factors and apoptosis (Fig. 6B) following vehicle and d-T3 treatment in CCSCs.
Figures 7A, 7B, 7C, and 7D show migration (Figs. 7A & 7B) and invasion (Figs. 7C & 7D) of CCSCs after treatment with vehicle, aspirin, d-T3, and aspirin + d-T3.
Figures 8A, 8B, 8C, and 8D show growth of cecal tumor volume (Figs. 8A & 8B) and liver metastasis score (Figs. 8C & 8D) in orthotopic model of CCSCs after treatment with vehicle, aspirin, d-T3, and aspirin + d-T3.
Figures 9A, 9B, 9C, and 9D show quantification of β-catenin in the nucleus and cytoplasm of CCSCs with confocal microscopy (Figs. 9A & 9B) after treatment with vehicle, aspirin, d-T3, and aspirin + d-T3. Figure 9C shows β-catenin degradation after treatment with d-T3 and pretreatment with proteosome inhibitor (PI), autophagy inhibitor (AI), calpain inhibitor (CP), and caspase 3 inhibitor (C3I) in CCSCs. Figure 9D shows western blot showing β-catenin, Cmyc, cyclin D1, and survivin after treatment with vehicle, aspirin, d-T3, and aspirin + d-T3 in CCSCs.
Figures 10A, 10B, 10C, and 10D show induction of apoptosis measured by flow cytometry (Fig. 10A) and confocal microscopy (Fig. 10B) in CCSCs after treatment with vehicle, aspirin, d-T3, and aspirin + d-T3. Effect of BID inhibitor on apoptosis in CCSCs after treatment with aspirin or d-T3 (Fig. 10C). Induction of t-BID in CCSCs after treatment with vehicle, aspirin, d-T3, and aspirin + d-T3 (Fig. 10D).
Figures 11A, 11B, 11C, 11D, 11E, and 11F show Heat map (Figs. 11A, 11B, 11C) and volcano plot (Figs. 11D, 11E, 11F) demonstrating upregulated, downregulated, and unchanged genes of CCSCs treated with (Figs. 11A, 11D) aspirin vs. vehicle, (Figs. 11B, 11E) d-T3 vs. vehicle, and (Figs. 11C, 11F) aspirin + d-T3 vs. vehicle.
Figure 12 shows differences between human pancreatic cancer tissues by stage based on immunohistochemistry of hCAS, showing the increasing presence of hCAS at various progressing stages prior and during pancreatic cancer development.
Figure 13 shows the basal expression of hCAS in several pancreatic cancer cell lines.
Figure 14 shows that the knock down of hCAS expression inhibits MiaPaCa-2 cell colony formation in soft agar.
Figures 15A and 15B show the hCAS knock down effect on (Fig. 15A) the pancreatic tumor volume of mice by hCAS SiRNA as compared to three controls, and (Fig. 15B) immunostains of hCAS, caspase-3, and Ki-67 in tumor.
Figure 16 shows the chemistry of preparing (1) delta-tocotrienol and (2) delta-tocopherol affinity gel.
Figure 17 shows the confirmatory work demonstrating that DADPA-d-T3 binds with hCAS in MiaPaCa-2 cells.

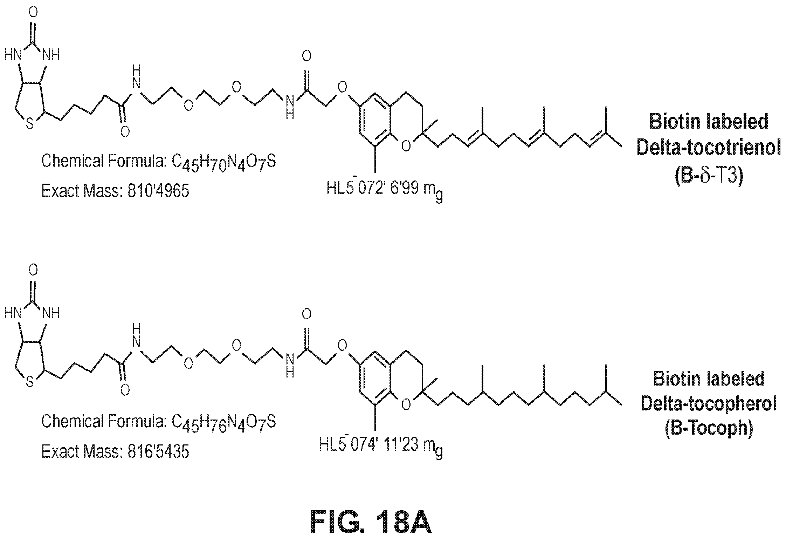

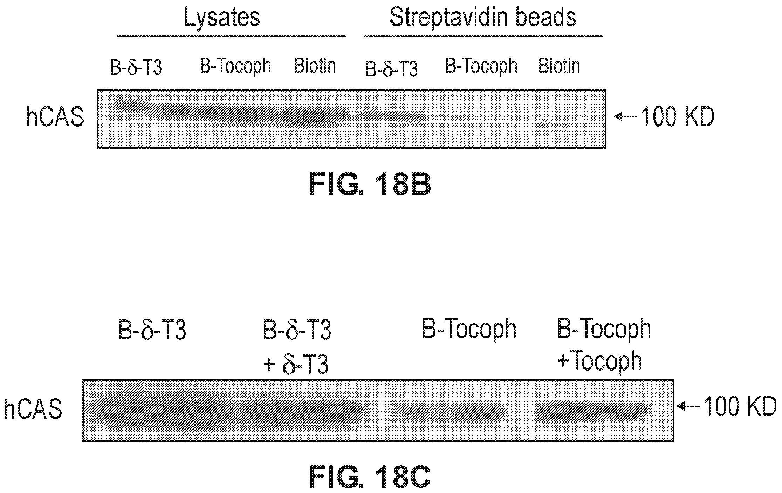

Figures 18A, 18B, and 18C show (Fig. 18A) the structures of biotin-labeled delta-tocotrienol and biotin-labeled delta-tocopherol, (Fig. 18B) their use to demonstrate the selective binding of the hCAS protein to biotin-d-T3 and not biotin-d-TOCOPH either directly from lysate or by using streptavidin-agarose beads, and (Fig. 18C) the competitive assay in MiaPaCa-2 cell lysate demonstrating that excess free d-T3 reduces hCAS binding to biotin-T3 and therefore competes for the d-T# binding site on the hCAS molecule whereas excess TOCOPH does not therefore does not compete with biotin-TOCOPH for binding to hCAS.
Figures 19A, and 19B show (Fig. 19A) the effect of d-T3 (50uM) and TOCOPH (50uM) on the presence of hCAS in the cystolic and nuclear fractions from MiaPaCa-2 cells incubated under starved and fed (FBS) conditions, and (Fig. 19B) the time-response of the effect of d-T3 (50uM) on hCAS protein expression in MiaPaCa-2 cells after 72-hour incubation.
Figure 20 shows the effect of biotin, biotinylated d-T3, biotinylated TOCOPH, free d-T3 and free TOCOPH on the viability of MiaPaCa-2 cells at increasing concentrations.
Figures 21A, and 21B show (Fig. 21A) the effect ofhCAS knock-down on MiaPaCa-2 cell proliferation, and (Fig. 21B) the effect of hCAS knock-down on MiaPaCa-2 cell proliferation by (1) control SiRNA, (2) control SiRNA + d-T3, (3) hCAS SiRNA, and (4) hCAS SiRNA + d-T3, showing that d-T3 has significant potency of its own and additive potency in inducing MiaPaCa-2 cell death as compared to hCAS SiRNA.
Figure 22 shows the effect of d-T3 on cell death in regular (Vector) and hCAS over-expressed (hCAS) MiaPaCa-2 cells.
Figures 23A and 23B show the effect of a d-T3 concentration escalation on the anchorage-independent growth and consequential colony formation of HPNE cells which are over-expressing hCAS, demonstrating a potent dose-dependent effect of d-T3 in reducing cell growth.

### DETAILED DESCRIPTION

Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### A. Definitions

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

In this specification and in the claims which follow, reference will be made to a number of terms which shall be defined to have the following meanings:

"Optional" or "optionally" means that the subsequently described event or circumstance may or may not occur, and that the description includes instances where said event or circumstance occurs and instances where it does not.

A "decrease" can refer to any change that results in a smaller amount of a symptom, disease, composition, condition, or activity. A substance is also understood to decrease the genetic output of a gene when the genetic output of the gene product with the substance is less relative to the output of the gene product without the substance. Also for example, a decrease can be a change in the symptoms of a disorder such that the symptoms are less than previously observed. A decrease can be any individual, median, or average decrease in a condition, symptom, activity, composition in a statistically significant amount. Thus, the decrease can be a 1, 2, 3, 4, 5, 6, 7, 8, 9, 10, 15, 20, 25, 30, 35, 40, 45, 50, 55, 60, 65, 70, 75, 80, 85, 90, 95, or 100% decrease so long as the decrease is statistically significant.

"Inhibit," "inhibiting," and "inhibition" mean to decrease an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.

By "reduce" or other forms of the word, such as "reducing" or "reduction," is meant lowering of an event or characteristic (e.g., tumor growth). It is understood that this is typically in relation to some standard or expected value, in other words it is relative, but that it is not always necessary for the standard or relative value to be referred to. For example, "reduces tumor growth" means reducing the rate of growth of a tumor relative to a standard or a control.

By "prevent" or other forms of the word, such as "preventing" or "prevention," is meant to stop a particular event or characteristic, to stabilize or delay the development or progression of a particular event or characteristic, or to minimize the chances that a particular event or characteristic will occur. Prevent does not require comparison to a control as it is typically more absolute than, for example, reduce. As used herein, something could be reduced but not prevented, but something that is reduced could also be prevented. Likewise, something could be prevented but not reduced, but something that is prevented could also be reduced. It is understood that where reduce or prevent are used, unless specifically indicated otherwise, the use of the other word is also expressly disclosed.

As used herein, "treatment," "treating" or the like refers to any process, action, application, therapy, or the like, wherein a subject, such as a human being, is subjected to medical aid with the object of curing a disorder (e.g. cancer) or improving the subject's condition, directly or indirectly. Treatment also refers to reducing incidence, alleviating symptoms, eliminating recurrence, preventing recurrence, preventing incidence, reducing the risk of incidence, improving symptoms, improving prognosis, or combinations thereof.

As used herein, a "therapeutically effective amount" means an amount of compound or compounds effective to prevent, reduce or inhibit a disorder (e.g., cancer) or symptom thereof in the subject being treated.

As used herein, "cancer stem cells" (or CSCs) are cancer cells (found within tumors or hematological cancers) that possess characteristics associated with normal stem cells, including the ability to give rise to all cell types found in a particular cancer sample. CSCs have been identified in various solid tumors, including brain, breast, colon, ovary, pancreas, prostate, melanoma, multiple myeloma, and non-melanoma skin cancer. Singh et al. (September 2003) Cancer Research. 63 (18): 5821-8; Al-Hajj M et al. PNAS (2003) 100 (7): 3983-8; O'Brien CA et al. (January 2007) Nature. 445 (7123): 106-10; Zhang S et al. (June 2008) Cancer Research. 68 (11): 4311-20; Li C et al., (February 2007) Cancer Research. 67 (3): 1030-7; Maitland NJ et al., (June 2008) Journal of Clinical Oncology. 26 (17): 2862-70; Schatton T et al. (January 2008) Nature. 451 (7176): 345-9; Civenni G et al., (April 2011) Cancer Research. 71 (8): 3098-109; Colmont CS et al., (January 2013) PNAS 110 (4): 1434-9. Markers used to identify normal stem cells are also commonly used for isolating CSCs from solid and hematological tumors. Markers that are frequently used for CSC isolation include: CD133 (also known as PROM1), CD44, ALDH1A1, CD34, CD24 and EpCAM (epithelial cell adhesion molecule, also known as epithelial specific antigen, ESA. Kim et al. (2017) Biochem. Moscow Suppl. Ser. B. 11 (1): 43-54.

Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

### B. Methods and Compositions

Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular δ-tocotrienol (d-T3) is disclosed and discussed and a number of modifications that can be made to a number of molecules including the δ-tocotrienol (d-T3) are discussed, specifically contemplated is each and every combination and permutation of d-T3 and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

d-T3, found in nuts, grains, palm oil, and other plant materials, having the formula 1 below, contains a lipophilic side chain with 16 carbons and 3 double bonds, as well as a chromanol ring with a phenolic group at position 6 and a methyl group at position 8. 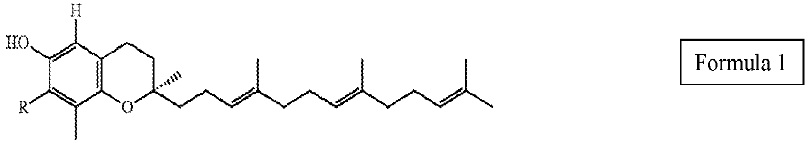 Wherein R is a hydrogen atom (H).

The phenolic group provides antioxidant activities. With the 5 group unmethylated, d-T3 has also strong activities in quenching reactive nitrogen species. Although a member of the vitamin E family, d-T3 has properties different from the commonly studied α-tocopherol (a-T), which has no double bond on the side chain and is trimethylated at positions 5, 7, and 8 of the chromanol ring. The structural difference makes these two compounds very different in cancer preventive activities. With inconsistent results in laboratory studies, clinical trials with large doses of a-T yielded disappointing results. On the other hand, d-T3 has shown promising cancer preventive effects. In terms of the mechanisms, most studies have reported inhibition of the Wnt pathway and activation of apoptosis in colon cancer; however, the primary targets remain to be identified. Of note is the reported inhibition of COX-1/2, 15-LOX, and dihydroceramide desaturase by d-T3/metabolites. The results also indicate that induction of apoptosis through β-catenin degradation can also be an important mechanism of d-T3 chemopreventive activity. In addition to the above-mentioned mechanisms, the quenching of reactive oxygen and nitrogen species and the activities of the side-chain degradation metabolites can be important. Because d-T3 is not effectively transported from the liver to the blood by a-T transport protein, the systemic bioavailability of d-T3 is much lower than a-T. The d-T3 in the liver undergoes side-chain degradation; the metabolites have been well identified and measured. The levels of these metabolites, carboxyethyl hydroxychroman (CEHC) and carboxymethylbutyl hydroxychroman (CMBHC), in blood and tissues can be higher than d-T3. Because d-T3 and its metabolites have direct contact with colon epithelial cells, the cells can take up the compound directly without going through the systemic route. Of note is that d-T3 produces the same metabolites as d-T. In the HPLC analysis, all forms of tocotrienols can be efficiently analyzed together with all forms of tocopherols and their metabolites. Therefore, the analysis of d-T3 in colon tumors and normal tissues can give an overall profile of d-T3 and its metabolites and an overall profile of vitamin E nutrition and metabolism in the colon as well as in colon neoplasia.

In one aspect, disclosed herein are methods of inhibiting, reducing, and/or preventing a cancer (such as, for example, recurrence of lung, colon, rectal, ovarian, pancreatic, and/or breast cancer) in a subject, cancer metastasis (including primary and secondary metastasis) in a subject, and/or cancer recurrence (such as, for example, recurrence of lung, colon, rectal, ovarian, pancreatic, and/or breast cancer) in a subject following surgical removal of a cancer or anti-cancer treatment (such as with an anti-cancer agent) of a cancer comprising administering to the subject a composition comprising a therapeutically effective amount of δ-tocotrienol (d-T3).

It is understood and herein contemplated that the disclosed methods of inhibiting, reducing, and/or preventing a cancer, cancer metastasis, and/or cancer recurrence in a subject comprising administering to the subject a composition comprising a therapeutically effective amount of δ-tocotrienol (d-T3) can be used to treat, inhibit, reduce, or prevent any disease where uncontrolled cellular proliferation occurs such as cancers. A non-limiting list of different types of cancers is as follows: lymphomas (Hodgkins and non-Hodgkins), leukemias, carcinomas, carcinomas of solid tissues, squamous cell carcinomas, adenocarcinomas, sarcomas, gliomas, high grade gliomas, blastomas, neuroblastomas, plasmacytomas, histiocytomas, melanomas, adenomas, hypoxic tumours, myelomas, AIDS-related lymphomas or sarcomas, metastatic cancers, or cancers in general.

A representative but non-limiting list of cancers that the disclosed compositions can be used to treat is the following: lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, lung cancers such as small cell lung cancer and non-small cell lung cancer, neuroblastoma/glioblastoma, ovarian cancer, skin cancer, liver cancer, melanoma, squamous cell carcinomas of the mouth, throat, larynx, and lung, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; glioblastoma; melanoma; sarcoma testicular cancer; colon cancer, rectal cancer, prostatic cancer, or pancreatic cancer.

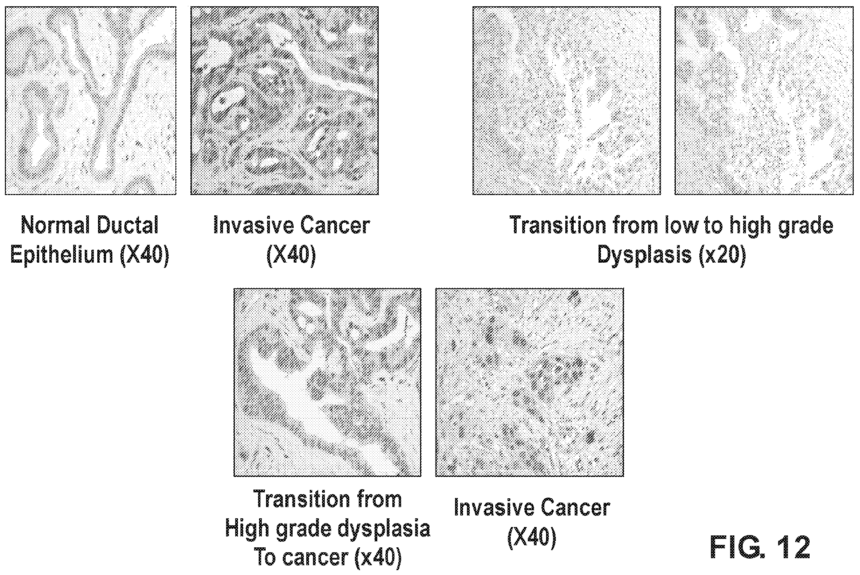

In certain embodiments, the cancer shares a common intracellular mechanism with colorectal cancer at the cancer stem cell (CSC) stage. These mechanisms on which CSCs depend include, but are not limited to, pathways activated or modulated through: hCAS protein (human cellular apoptosis susceptibility protein, also known as CSE1L [Pimiento et al; Am.J.Pathol.; Vol.186 No.10; Oct.2016]) (as exemplified in Example 5 and Figures 12-23); XPO-2; (exportin-2); c-FLIP, also identified as CFLAR [Francois et al, Cancer Cell International (2019) 19-189]; and EGR-1/Bax [Wang et al; J.Nutr.Biol., Vol.26 (2015) 797-807].

hCAS plays a pivotal role in exporting out of the nucleus into the cytosol importin-alpha, a key component of the Importin alpha/importin beta/RanGTPase complex that imports NLS-containing proteins from the cytoplasm to the nucleus. hCAs is over expressed in tumors compared to normal and its high levels correlate with tumor grade, aggressiveness, invasion and metastasis and poor patient outcomes ([Behrens et al.; Apoptosis. 2003;8(1):39-44]; [Chang et al.; Annals of diagnostic pathology. 2012;16(5):362-8]; [Sillars-Hardebol et al.; Cellular oncology (Dordrecht). 2012;35(4):293-300]; [Stella Tsai et al.; The American journal of pathology. 2010;176(4):1619-28]; [Sugiura et al.; Cancer biology & therapy. 2008;7(2):285-92]; [Tai et al.; Journal of experimental & clinical cancer research : CR. 2010;29:110]; [Tung et al.; Cancer epidemiology, biomarkers & prevention. 2009;18(5):1570-7]). The discovery that delta-tocotrienol binds to and reduces the levels of hCAS (Example 5) and that this biochemical activity is important for its antiproliferative and pro apoptotic effects is novel. This is the first identification of a molecule with high affinity for hCAS and that induces the degradation of hCAS in cells. The observation that hCAS expression is increased in premalignant and malignant pancreatic cancer cells is also novel. The concept of targeting the increased expression of hCAS and thereby the nuclear cytoplasmic transport process for chemoprevention of pancreatic cancer (Example 5) is innovative with broad implications for the chemoprevention of other cancers such as colorectal cancer which also has increased expression of hCAS in premalignant lesions (colon polyps).

In addition, delta-tocotrienol inhibits CSCs viability, survival, self-renewal (spheroid formation), expression of pluripotent transcription factors (nanog, Oct4 and Sox2), organoids formation and/or Wnt/β-catenin signaling. In addition, delta-tocotrienol inhibits the migration, invasion, inflammation (NF-kB), angiogenesis (VEGF) and/or metastasis (MMP9) in CSCs. These processes are important for tumor metastases. Moreover d-T3 induces apoptosis (TUNEL, Annexin V, cleaved caspase 3 and cleaved PARP) in CSCs and CSCs-derived spheroids and organoids. Finally, in a new orthotopic (cecum-injected CCSCs) xenograft model of metastasis, we show that d-T3 significantly retards the CSCs-derived tumor growth (Ki-67), inhibits inflammation (NF-kB), angiogenesis (VEGF and CD31), β-catenin, and induced apoptosis (C-PARP) in tumor tissues and inhibits liver metastasis of CRC. As CSCs of other cancer types share common intracellular mechanisms with CRC CSCs, These mechanisms, which are shared by CSCs of cancer types other than CRC, demonstrate the unique ability to use of delta-tocotrienol for chemoprevention/treatment of a broad range of cancer type metastases either alone or in combination with other chemotherapeutic drugs.

The term "subject" refers to any individual who is the target of administration or treatment. The subject can be a vertebrate, for example, a mammal. In one aspect, the subject can be human, non-human primate, bovine, equine, porcine, canine, or feline. The subject can also be a guinea pig, rat, hamster, rabbit, mouse, or mole. Thus, the subject can be a human or veterinary patient. The term "patient" refers to a subject under the treatment of a clinician, e.g., physician.

The term "therapeutically effective" refers to the amount of the composition used is of sufficient quantity to ameliorate one or more causes or symptoms of a disease or disorder. Such amelioration only requires a reduction or alteration, not necessarily elimination.

The term "composition" refers to the form in which the delta-tocotrienol is administered to the subject. The composition can, for example, be contained in a tablet (absorbed to a carrier), a hard gelatin capsule, or softgel capsule. In one embodiment, the composition, an oily concentrate or isolate of delta-tocotrienol, is made up predominantly of delta-tocotrienol, preferably more than 50% delta-tocotrienol, more preferably more than 60%, more preferably more than 70%, more preferably more than 80%, more preferably more than 85%, more preferably more than 90%, more preferably more than 93%, more preferably more than 95%, more preferably more than 96%, more preferably more than 97%, more preferably more than 97.5%, more preferably more than 98%, more preferably more than 98.5%, most preferably more than 99% delta-tocotrienol.

It is commonly known that in biology, structurally similar compounds may interfere with each other's activity, for instance, by blocking access to certain receptor or enzymatic sites. For example, alpha-tocopherol (a-T) is known to interfere with one certain mode-of-action of delta-tocotrienol (d-T3). Thus, in certain embodiments, the delta-tocopherol is isolated from other compounds with similar structural and biochemical features, such as alpha-tocotrienol, beta-tocotrienol, and/or gamma-tocotrienol, as well as alpha-tocopherol, beta-tocopherol, gamma-tocopherol and delta-tocopherol,that are present in palm oil and typical extracts, such as vitamin E preparations or tocotrienol preparations. The main limitation of such commercially available preparations, is that the commercially available vitamin E and tocotrienol preparations are crude mixtures that are not pure enough to circumvent the interference issues.

In one embodiment therefore, the ratio (d-T3: a-T) of delta-tocotrienol as compared to alpha-tocopherol in the composition is at least 10, more preferably at least 20, more preferably at least 30, more preferably at least 40, more preferably at least 50, more preferably at least 60, more preferably at least 70, more preferably at least 80, more preferably at least 90, more preferably at least 100, more preferably at least 125, more preferably at least 150, more preferably at least 200, more preferably at least 300, more preferably at least 500, more preferably at least 700, more preferably at least 1000, more preferably at least 2000, most preferably at least 4000.

In another embodiment therefore, the ratio (d-T3: d-T) of delta-tocotrienol as compared to delta-tocopherol in the composition is at least 10, more preferably at least 20, more preferably at least 30, more preferably at least 40, more preferably at least 50, more preferably at least 60, more preferably at least 70, more preferably at least 80, more preferably at least 90, more preferably at least 100, more preferably at least 125, more preferably at least 150, more preferably at least 200, more preferably at least 300, more preferably at least 500, more preferably at least 700, more preferably at least 1000, more preferably at least 2000, most preferably at least 4000.

In yet another embodiment, the ratio (d-T3 : a-T3) of delta-tocotrienol as compared to alpha-tocotrienol (b-T3) in the composition is at least 10, more preferably at least 20, more preferably at least 30, more preferably at least 40, more preferably at least 50, more preferably at least 60, more preferably at least 70, more preferably at least 80, more preferably at least 90, more preferably at least 100, more preferably at least 125, more preferably at least 150, more preferably at least 200, more preferably at least 300, more preferably at least 500, more preferably at least 700, more preferably at least 1000, most preferably at least 2000.

In a further embodiment, the ratio (d-T3 : b-T3) of delta-tocotrienol as compared to beta-tocotrienol (b-T3) in the composition is at least 10, more preferably at least 20, more preferably at least 30, more preferably at least 40, more preferably at least 50, more preferably at least 60, more preferably at least 70, more preferably at least 80, more preferably at least 90, more preferably at least 100, more preferably at least 125, more preferably at least 150, more preferably at least 200, more preferably at least 300, more preferably at least 500, more preferably at least 700, more preferably at least 1000, most preferably at least 2000.

In another further embodiment, the ratio (d-T3 : g-T3) of delta-tocotrienol as compared to gamma-tocotrienol (g-T3) in the composition is at least 10, more preferably at least 20, more preferably at least 30, more preferably at least 40, more preferably at least 50, more preferably at least 60, more preferably at least 70, more preferably at least 80, more preferably at least 90, more preferably at least 100, more preferably at least 125, more preferably at least 150, more preferably at least 200, more preferably at least 300, more preferably at least 500, more preferably at least 700, more preferably at least 1000, most preferably at least 2000.

Finally, in another embodiment, the ratio (d-T3 : ccT) of delta-tocotrienol as compared to all tocopherols (i.e., the sum of a-T, b-T, g-T, and d-T) collectively (ccT) in the composition is at least 10, more preferably at least 20, more preferably at least 30, more preferably at least 40, more preferably at least 50, more preferably at least 60, more preferably at least 70, more preferably at least 80, more preferably at least 90, more preferably at least 100, more preferably at least 125, more preferably at least 150, more preferably at least 200, more preferably at least 300, more preferably at least 500, more preferably at least 700, more preferably at least 1000, more preferably at least 2000, most preferably at least 4000.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

In one aspect, it is understood and herein contemplated that δ-tocotrienol (d-T3) can be used to inhibit, reduce, and/or prevent a cancer. That is, the d-T3 can be administered as a chemopreventive to a subject at risk of developing a cancer. The chemopreventative activity can be accomplished with or without the further administration of aspirin or another NSAID or COX-2 inhibitor to the subject.

As noted above, it is intended herein that the disclosed methods of inhibiting, reducing, and/or preventing cancer metastasis and/or recurrence as well as anti-cancer maintenance methods can follow any therapeutic treatment of a cancer including, but not limited surgical, radiological, and/or pharmaceutical treatments of a cancer. As used herein, "surgical treatment" refers to tumor resection of the tumor by any means known in the art. Similarly, "pharmaceutical treatment" refers to the administration of any anti-cancer agent known in the art including, but not limited to those agents in the following LIST A-C: Abemaciclib, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin Chlorambucil), Amifostine, Aminolevulinic Acid, Anastrozole, Aprepitant, Aredia (Pamidronate Disodium), Arimidex (Anastrozole), Aromasin (Exemestane),Arranon (Nelarabine), Arsenic Trioxide, Arzerra (Ofatumumab), Asparaginase Erwinia chrysanthemi, Atezolizumab, Avastin (Bevacizumab), Avelumab, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin) , Bevacizumab, Bexarotene, Bexxar (Tositumomab and Iodine 1131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Campath (Alemtuzumab), Camptosar , (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride , EPOCH, Erbitux (Cetuximab), Eribulin Mesylate, Erivedge (Vismodegib), Erlotinib Hydrochloride, Erwinaze (Asparaginase Erwinia chrysanthemi) , Ethyol (Amifostine), Etopophos (Etoposide Phosphate), Etoposide, Etoposide Phosphate, Evacet (Doxorubicin Hydrochloride Liposome), Everolimus, Evista , (Raloxifene Hydrochloride), Evomela (Melphalan Hydrochloride), Exemestane, 5-FU (Fluorouracil Injection), 5-FU (Fluorouracil-Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil-Topical), Fluorouracil Injection, Fluorouracil--Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride), Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride , Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and , Hyaluronidase Human, ,Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq , (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil--Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Vandetanib, VAMP, Varubi (Rolapitant Hydrochloride), Vectibix (Panitumumab), VelP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), and/or Zytiga (Abiraterone Acetate). Also contemplated herein as part of List A-C are chemotherapeutics that are PD1/PDL1 blockade inhibitors (such as, for example, lambrolizumab, nivolumab, pembrolizumab, pidilizumab, BMS-936559, Atezolizumab, Durvalumab, or Avelumab).

As described above, the δ-tocotrienol comprising compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

Pharmaceutical carriers are known to those skilled in the art. These most typically would be standard carriers for administration of drugs to humans, including solutions such as sterile water, saline, and buffered solutions at physiological pH. The δ-tocotrienol comprising compositions can be administered intramuscularly or subcutaneously. Other compounds will be administered according to standard procedures used by those skilled in the art.

Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

The δ-tocotrienol comprising compositions may be administered orally, parenterally (e.g., intravenously), by intramuscular injection, by intraperitoneal injection, transdermally, extracorporeally, topically or the like, including topical intranasal administration or administration by inhalant.

As used herein, "topical intranasal administration" means delivery of the δ-tocotrienol comprising compositions into the nose and nasal passages through one or both of the nares and can comprise delivery by a spraying mechanism or droplet mechanism, or through aerosolization of the nucleic acid or vector. Administration of the δ-tocotrienol comprising compositions by inhalant can be through the nose or mouth via delivery by a spraying or droplet mechanism. Delivery can also be directly to any area of the respiratory system (e.g., lungs) via intubation. The exact amount of the composition comprising δ-tocotrienol required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

Compositions comprising δ-tocotrienol for oral administration include powders or granules, suspensions or solutions in water or non-aqueous media, capsules (hard gelatin and softgel capsules), sachets, or tablets. Thickeners, flavorings, diluents, emulsifiers, dispersing aids or binders may be desirable.

Some of the compositions comprising δ-tocotrienol may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

Effective dosages and schedules for administering the compositions comprising δ-tocotrienol may be determined empirically, and making such determinations is within the skill in the art. The dosage ranges for the administration of the compositions comprising δ-tocotrienol are those large enough to produce the desired effect in which the symptoms of the disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above. In one aspect, the daily dose of compositions comprising d-T3 can be between about 5µg and 6400mg, preferably between about 5 and 6000mg, more preferably between about 100 and 2000mg, more preferably between about 400 and 3200mg, most preferably between about 800 and 1600mg. For example, the daily dose of d-T3 can be 5, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500, 550, 600, 650, 700, 750, 800, 850, 900, or 950µg, and 1, 2, 3, 4, 5, 6, 7, 8, 9, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, 500, 550, 600, 650, 700, 750, 800, 850, 900, 950, 1000, 1200, 1400, 1500, 1600, 1800, 2000, 2200, 2400, 2500, 2600, 2800, 3000, or 3200mg, or any ranges in between these doses. It is understood and herein contemplated that the effective dose can also be expressed in molar concentration as measured in blood levels or in the target organ or tissue. In one aspect, the effective daily dose of d-T3 can comprise levels of 5, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400µM.

In one aspect, it is understood and herein contemplated that the compositions comprising d-T3 can be administered as a single dose or multiple times in a single day to achieve the daily dosage. In one aspect, disclosed herein are methods of inhibiting a cancer recurrence (such as, for example, recurrence of lung, colon, rectal, ovarian, pancreatic, and/or breast cancer) and/or maintaining a post cancer treatment therapy in a subject following surgical removal or anti-cancer treatment of a cancer; methods of preventing a cancer (such as, for example, recurrence of lung, colon, rectal, ovarian, pancreatic, and/or breast cancer); and/or methods of inhibiting, reducing and/or preventing metastasis of a cancer (such as, for example, recurrence of lung, colon, rectal, ovarian, pancreatic, and/or breast cancer), said methods comprising administering to the subject a therapeutically effective amount of a composition comprising δ-tocotrienol (d-T3), wherein the d-T3 composition is administered 1, 2, 3, 4, 5, 6, 7, 8, 9, 10, 11, 12, 13, 14, 15, 16, 17, 18, 19, 20, 21, 22, 23, 24 times per day.

It is further understood and herein contemplated that the compositions comprising d-T3 can be formulated to have prolonged release of d-T3 or the effective dosage can be administered less frequently than daily administration. Accordingly, disclosed herein are methods of inhibiting a cancer recurrence (such as, for example, recurrence of lung, colon, rectal, ovarian, pancreatic, and/or breast cancer) and/or maintaining a post cancer treatment therapy in a subject following surgical removal or anti-cancer treatment of a cancer; methods of preventing a cancer (such as, for example, recurrence of lung, colon, rectal, ovarian, pancreatic, and/or breast cancer); and/or methods of inhibiting, reducing and/or preventing metastasis of a cancer (such as, for example, recurrence of lung, colon, rectal, ovarian, pancreatic, and/or breast cancer), said methods comprising administering to the subject a therapeutically effective amount of a composition comprising δ-tocotrienol (d-T3), wherein the d-T3 composition is administered one time every 2, 3, 4, 6, 8, 12, 18, 24, 36, 48, 60, 72 hours, 4, 5, 6, 7, 10, 14 days, 3, 4, 5, 6, 7, 8 weeks, 3, 4, 5, 6, 7, 8, 9, 10, 11, 12 months.

While a single administration of d-T3 for the further prevention of cancer recurrence, first occurrence, metastasis, and/or post-treatment maintenance would be ideal, it is understood and herein contemplated that to inhibit cancer recurrence, first occurrence, metastasis, and/or post-treatment maintenance the disclosed d-T3 composition may need to be administered for an extended period of time or the remaining life of the subject. Thus, in one aspect, disclosed herein are methods of inhibiting a cancer recurrence (such as, for example, recurrence of lung, colon, rectal, ovarian, pancreatic, and/or breast cancer) and/or maintaining a post cancer treatment therapy in a subject following surgical removal or anti-cancer treatment of a cancer; methods of preventing a cancer (such as, for example, recurrence of lung, colon, rectal, ovarian, pancreatic, and/or breast cancer); and/or methods of inhibiting, reducing and/or preventing metastasis of a cancer (such as, for example, recurrence of lung, colon, rectal, ovarian, pancreatic, and/or breast cancer), said methods comprising administering to the subject a therapeutically effective amount of a composition comprising δ-tocotrienol (d-T3), wherein the d-t3 composition is administered for at least 1, 2, 3, 4, 5, 6, 7, 8, 9, 10, 11, 12, 13, 14 days, 3, 4, 5, 6, 7, 8 weeks, 2, 3, 4, 5, 6, 7, 8, 9, 10, 11, 12, 18 months, 2, 3, 4, 5, 6, 7, 8, 9, 10, or more years.

### The use of aspirin or other NSAIDs in combination with d-T3 for CRC prevention

The landmark endorsement by the US Preventive Services Task Force of aspirin for primary prevention of CRC in 2016 implies that, from now on, aspirin can be the backbone of any novel strategy to prevent CRC. The data presented herein clearly demonstrate that d-T3 augments aspirin activity in several cell and animal models of CRC. Both d-T3/metabolites and aspirin have been proposed to inhibit COX-1/2. This only produces an additive effect for the two agents. The enhanced β-catenin degradation induced by d-T3, in combination with the additive effect in the inhibition of COX-1/2, can produce synergy. It is well established that the initiation of CRC predominantly involves activation of the Wnt pathway, mostly due to APC mutation, resulting in the accumulation in β-catenin in the nucleus. Another important driving force is the promotion of tumorigenesis by inflammation, which involves activation of the redox-sensitive transcription factor NF-κB and COX-2. The suppression of NF-κB by the antioxidant property of d-T3 (or the inhibition of 15-LOX or alteration of sphingolipid metabolism by d-T3), in combination with the additive effect in the inhibition of COX-1/2, can also produce synergistic effects.

Accordingly, disclosed herein are methods of inhibiting, reducing, and/or preventing a cancer (such as, for example, recurrence of lung, colon, rectal, ovarian, pancreatic, and/or breast cancer) in a subject, cancer metastasis (including primary and secondary metastasis) in a subject, and/or cancer recurrence (such as, for example, recurrence of lung, colon, rectal, ovarian, pancreatic, and/or breast cancer) as well as methods of post cancer treatment maintenance in a subject following surgical removal of a cancer or anti-cancer treatment (such as with an anti-cancer agent) of a cancer comprising administering to the subject a therapeutically effective amount of a composition comprising δ-tocotrienol (d-T3), further comprising administering to the subject aspirin or another NSAID or COX-2 inhibitor, including but not limited to: Diflunisal; Salicylic acid and its salts; Salsalate; Propionic acid derivatives; Ibuprofen; Dexibuprofen; carprofen; Naproxen; Fenoprofen; Ketoprofen; Dexketoprofen; Flurbiprofen; Oxaprozin; Loxoprofen; Indomethacin; tolmetin; Sulindac; Etodolac; Ketorolac; Diclofenac; Aceclofenac; nabumetone; Piroxicam; Meloxicam; Tenoxicam; Droxicam; Lornoxicam; Isoxicam; Phenylbutazone; Mefenamic acid; Meclofenamic acid; Flufenamic acid; Tolfenamic acid; Nimesulide; Celecoxib; Rofecoxib; Valdecoxib; Parecoxib; Lumiracoxib; Etoricoxib; and Firocoxib..

The usage of aspirin and appropriate dosage can be determined empirically for the subject by a physician. Nevertheless, disclosed herein are methods of inhibiting a cancer recurrence comprising administering to the subject a therapeutically effective amount of a composition comprising δ-tocotrienol (d-T3) and aspirin, wherein the aspirin daily dosage is between about 50 and 1000mg, preferably between about 50 and 500mg, more preferably between about 50 and 325mg. For example, disclosed herein are methods wherein the effective daily dosage of aspirin comprises 50, 55, 60, 65, 70, 70, 71, 72, 73, 74, 75, 76, 77, 78, 79, 80, 81, 82, 83, 84, 85, 90, 95, 100, 125, 150, 175, 200, 225, 250, 275, 300, 325, 350, 375, 400, 425, 450, 475, 500, 550, 600, 650, 700, 750, 800, 850, 900, 950, or 1000mg. It is understood and herein contemplated that the use of d-T3 in combination with aspirin lowers the effective dosage (i.e., increases the efficacy) of aspirin. Equivalent doses of other NSAIDs and COX-2 inhibitors are also included and will be apparent to those skilled in the art.

In one aspect, it is understood and herein contemplated that the disclosed methods of inhibiting, reducing, and/or preventing a cancer or cancer metastasis in a subject, and/or cancer recurrence (such as, for example, recurrence of lung, colon, rectal, ovarian, pancreatic, and/or breast cancer) or post cancer treatment maintenance in a subject following surgical removal of a cancer or anti-cancer treatment (such as with an anti-cancer agent including, but not limited to chemotherapeutics, anti-cancer antibodies, immunotherapies (including CAR T cells, checkpoint inhibitors, and TILs), and radiation) of a cancer comprising administering to the subject a therapeutically effective amount of a composition comprising δ-tocotrienol (d-T3) can further comprise the continued administration of an anti-cancer agent. The anti-cancer agent can comprise any anti-cancer agent known in the art including, but not limited to antibodies, tumor infiltrating lymphocytes, checkpoint inhibitors, dendritic cell vaccines, anti-tumor vaccines, immunotherapy, and chemotherapeutic agents. In one aspect, the anti-cancer agent can include, but is not limited to any of the agents included on LIST A-C (see paragraph 75 above).

The combination of δ-tocotrienol and anti-cancer agent can be formulated in the same composition or formulated and administered separately. Where separate, the composition comprising δ-tocotrienol can be administered before, after, or concurrently with the chemotherapeutic agent. Administration of δ-tocotrienol can be administered prophylactically or therapeutically for the inhibition, treatment, reduction, and/or prevention of a cancer or metastasis or prophylactically or therapeutically for the inhibition, treatment, reduction, and/or prevention of a cancer recurrence following therapeutic treatment of a cancer (including resection, radiation, immunotherapy, and/or chemotherapy). It is understood that he use of the δ-tocotrienol provides the advantage of increasing the efficacy of any anti-cancer therapy and thus has the added benefit o flowering dosages of companion therapies and thus can also limit unwanted side effects of those therapies.

### Samples

The diagnostic methods described herein involve analysis of hCAS expression levels in cancerous or precancerous cells. These cancerous or precancerous cells may be found in a biological sample, such as blood or fractions thereof, such as serum or plasma, urine, or tissue, including for example, a primary tumor tissue or a biopsy tissue. Nucleic acids or polypeptides may be isolated from the cells prior to detecting hCAS expression levels.

In one embodiment, the biological sample comprises tissue and is obtained through a biopsy. In another embodiment, the biological sample is blood or a fraction thereof, such as plasma or serum. In certain embodiments, the biological sample is blood or a fraction thereof, such as plasma or serum, and contains circulating tumor cells that have detached from a primary tumor.

### Controls

The control may be any suitable reference that allows evaluation of hCAS expression levels. In certain embodiments, the control is a biological sample comprising non-cancerous cells from a matched subject, or a pool of such samples. Thus, for instance, the control can be a sample from the same subject that is analyzed simultaneously or sequentially with the test sample, or the control can be the average hCAS expression level in a pool of biological samples from healthy subjects or otherwise known to be non-cancerous. Alternatively, the control can be defined by mRNA copy numbers of other genes in the sample, such as housekeeping genes (e.g., PBGD or GAPDH) or other genes that can be used to normalize gene expression levels. In certain embodiments, the control is a predetermined "cut-off" or threshold value of absolute expression. Thus, the control can be embodied, for example, in a pre-prepared microarray used as a standard or reference, or in data that reflects the hCAS expression profile in a sample or pool of non-cancerous samples, such as might be part of an electronic database or computer program.

Overexpression of hCAS can be determined by any suitable method, such as by comparing hCAS expression in a test sample with a control (e.g., a positive or negative control or threshold value). A control can be provided as previously discussed. Regardless of the method used, overexpression can be defined as any level of expression greater than or less than the level of expression of an appropriate control. By way of further illustration, overexpression can be defined as expression that is at least about 1.2-fold, 1.5-fold, 2-fold, 2.5-fold, 4-fold, 5-fold, 10-fold, 20-fold, 50-fold, 100-fold higher or even greater expression as compared to the control.

### Diagnostic Methods

As described herein, overexpression of hCAS can be used to identify subjects at risk for developing cancer metastasis or recurrence.

Thus, one aspect is directed to a method of determining if a subject is at risk for developing cancer metastasis or recurrence, the method comprising assaying a biological sample from the subject to determine a level of hCAS expression in the sample, wherein an increased expression level of hCAS as compared to a control indicates that the subject has an increased risk to develop cancer metastasis or recurrence. In the event of such a result, the methods may include one or more of the following steps: informing the subject that they are likely to have metastasis or cancer recurrence; confirmatory biospy; and/or treating the subject for metastasis or cancer recurrence. In certain embodiments, the cancer includes is lung cancer, ovarian cancer, breast cancer, rectal cancer, sarcoma, liver cancer, pancreatic or colon cancer. In certain embodiments, the cancer is pancreatic or colon cancer. In certain embodiments, the cancer comprises cancer stem cells (CSCs).

Determining the expression levels of hCAS comprises measuring or detecting any hCAS nucleic acid transcript (e.g., mRNA or cDNA) or the protein encoded thereby. Typically, gene expression can be detected or measured on the basis of mRNA or cDNA levels, although protein levels also can be used when appropriate. Any quantitative or qualitative method for measuring mRNA levels, cDNA, or protein levels can be used. Suitable methods of detecting or measuring mRNA or cDNA levels include, for example, Northern Blotting, microarray analysis, or a nucleic acid amplification procedure, such as reverse-transcription PCR (RT-PCR) or real-time RT-PCR, also known as quantitative RT-PCR (qRT-PCR). Such methods are well known in the art. *See e.g*., Sambrook et al., Molecular Cloning: A Laboratory Manual, 4th Ed., Cold Spring Harbor Press, Cold Spring Harbor, N.Y., 2012. Other techniques include digital, multiplexed analysis of gene expression, such as the nCounter^{®} (NanoString Technologies, Seattle, WA) gene expression assays, which are further described in US20100112710 and US20100047924.

Detecting a nucleic acid of interest generally involves hybridization between a target nucleic acid and a probe. Sequences of the hCAS gene and mRNA encoded thereby are known. Therefore, one of skill in the art can readily design hybridization probes for detecting hCAS. *See*, *e*.*g*., Sambrook et al., Molecular Cloning: A Laboratory Manual, 4th Ed., Cold Spring Harbor Press, Cold Spring Harbor, N.Y., 2012. The probe should be substantially specific for hCAS to avoid any cross-hybridization and false positives.

Alternatively or additionally, expression levels of hCAS can be determined at the protein level, meaning that levels of hCAS proteins are measured. Several methods and devices are known for determining levels of proteins including immunoassays, such as described, for example, in U.S. Pat. Nos. 6,143,576; 6,113,855; 6,019,944; 5,985,579; 5,947,124; 5,939,272; 5,922,615; 5,885,527; 5,851,776; 5,824,799; 5,679,526; 5,525,524; 5,458,852; and 5,480,792, each of which is hereby incorporated by reference in its entirety. These assays may include various sandwich, competitive, or non-competitive assay formats, to generate a signal that is related to the presence or amount of a protein of interest. Any suitable immunoassay may be utilized, for example, lateral flow, enzyme-linked immunoassays (ELISA), radioimmunoassays (RIAs), competitive binding assays, and the like.

Although immunoassays have been used for the identification and quantification of proteins, recent advances in mass spectrometry (MS) techniques have led to the development of sensitive, high-throughput MS protein analyses. The MS methods can be used to detect low abundant proteins in complex biological samples. For example, it is possible to perform targeted MS by fractionating the biological sample prior to MS analysis. Common techniques for carrying out such fractionation prior to MS analysis include, for example, two-dimensional electrophoresis, liquid chromatography, and capillary electrophoresis. Selected reaction monitoring (SRM), also known as multiple reaction monitoring (MRM), has also emerged as a useful high-throughput MS-based technique for quantifying targeted proteins in complex biological samples.

The methods may be used to assess the need for therapy or to monitor a response to a therapy (e.g., disease-free recurrence following surgery or other therapy), and, thus may include an additional step of treating a subject. In certain embodiments, the methods further comprise treating the subject for cancer metastasis or recurrence. In certain embodiments, the subject has previously undergone cancer therapy (e.g., standard of care cancer therapy) for a primary cancer, such as surgery, chemotherapy, or radiation or any other cancer treatment prior to treating the subject for cancer metastasis or recurrence. In certain embodiments, the subject receives maintenance therapy for cancer metastasis or recurrence after receiving cancer therapy for the primary cancer. In certain embodiments, the subject is treated after being identified as having an increased risk to develop metastasis or cancer recurrence but before metastasis or cancer recurrence has been confirmed, for example, by a biopsy. In other embodiments, the subject receives treatment after metastasis or recurrence has been confirmed, for example, by a biopsy.

A related aspect is directed to a method of inhibiting metastasis of a cancer, inhibiting a cancer recurrence, or maintenance therapy in a subject following cancer therapy, the method comprising administering to the subject a composition comprising a therapeutically effective amount of a compound that reduces hCAS levels in cancerous or pre-cancerous tissues, wherein prior to the administering step, the subject was identified as having increased levels of hCAS expression relative to a control.

In certain embodiments, the treating step comprises administering a therapeutically effective amount of a compound that reduces hCAS levels in cancerous or pre-cancerous tissues. In certain embodiments, the compound comprises a nucleic acid inhibitor molecule that reduces hCAS mRNA expression levels . In certain embodiments, the compound comprises δ-tocotrienol (d-T3). In certain embodiments, d-T3 is administered in combination with a nucleic acid inhibitor molecule that reduces hCAS mRNA expression levels.

### Nucleic Acid Inhibitor Molecules

The term "nucleic acid inhibitor molecule" refers to an oligonucleotide molecule that reduces or eliminates the expression of a target gene wherein the oligonucleotide molecule contains a region that specifically targets a sequence in the target gene mRNA. Typically, the targeting region of the nucleic acid inhibitor molecule comprises a sequence that is sufficiently complementary to a sequence on the target gene mRNA to direct the effect of the nucleic acid inhibitor molecule to the specified target gene. The nucleic acid inhibitor molecule may include ribonucleotides, deoxyribonucleotides, and/or modified nucleotides.

Various oligonucleotide structures have been used as nucleic acid inhibitor molecules, including single stranded and double stranded oligonucleotides, and any of these various oligonucleotides can be modified to include one or more glutathione-sensitive nucleotides as described herein.

In certain embodiments, the nucleic acid inhibitor molecule is a double-stranded RNAi inhibitor molecule comprising a sense (or passenger) strand and an antisense (or guide strand). A variety of double stranded RNAi inhibitor molecule structures are known in the art. For example, early work on RNAi inhibitor molecules focused on double-stranded nucleic acid molecules with each strand having sizes of 19-25 nucleotides with at least one 3'-overhang of 1 to 5 nucleotides (see, e.g., U.S Patent No. 8,372,968). Subsequently, longer double-stranded RNAi inhibitor molecules that get processed *in vivo* by the Dicer enzyme to active RNAi inhibitor molecules were developed (see, e.g., U.S. Patent No. 8,883,996).

In certain embodiments, the nucleic acid inhibitor molecule is a single-stranded nucleic acid inhibitor molecule comprising at least one nucleotide having a glutathione-sensitive moiety, as described herein. Single stranded nucleic acid inhibitor molecules are known in the art. For example, recent efforts have demonstrated activity of ssRNAi inhibitor molecules (see, e.g., Matsui et al., Molecular Therapy, 2016,24(5):946-55. And, antisense molecules have been used for decades to reduce expression of specific target genes. Pelechano and Steinmetz, Nature Review Genetics, 2013,14:880-93. A number of variations on the common themes of these structures have been developed for a range of targets. Single stranded nucleic acid inhibitor molecules include, for example, conventional antisense oligonucleotides, microRNA, ribozymes, aptamers, antagomirs, and ssRNAi inhibitor molecules, all of which are known in the art.

### C. Examples

The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### Example 1: d-T3 administration augments aspirin activity, suppresses adenoma formation, and induces apoptosis in the intestinal stem cells of ApcMinl+ mice.

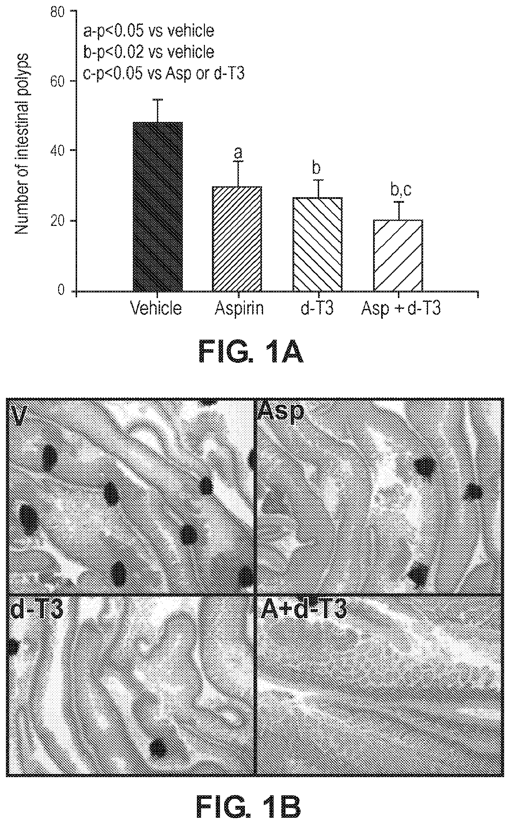

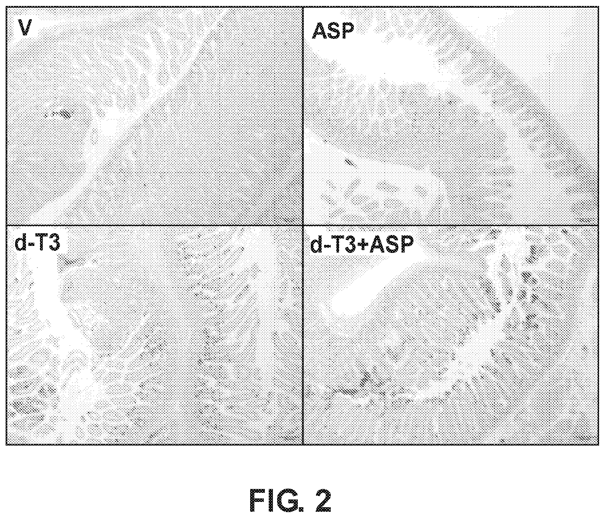

Although substantial preclinical evidence exists regarding the preventive activity of d-T3 and g-T3 in various types of cancers, there is no report of d-T3 prevention of colon cancer despite evidence of activity in colon cell lines and xenograft models. To study the role of d-T3 in colon cancer prevention, the effects of d-T3 were compared to aspirin and the combination of d-T3 and aspirin in *Apc*Min*l*+ mice (Fig. 1). d-T3 significantly suppresses adenoma formation as effectively as aspirin and significantly augments aspirin activity. Treatment with aspirin alone reduced adenoma formation by 38%, and treatment with d-T3 alone reduced adenoma formation by 44%, while treatment with the combination of aspirin and d-T3 reduced adenoma formation by 58%. Furthermore, as shown in Fig. 2, d-T3 markedly induced apoptosis detected by caspase 3 staining. d-T3 treatment resulted in caspase 3 staining all along the small intestinal crypts of mice, while treatment with vehicle or aspirin alone revealed minimal to no staining in intestinal cells. The combination of aspirin and d-T3 demonstrated marked staining, mostly along the apical and differentiated side of the intestinal crypts. This result indicates that d-T3 eliminated *APC*-mutant cancer initiating cells in the base of the intestinal crypts and transformed differentiated cells along the intestinal crypts. These results are consistent with the *in vitro* results and prompted the testing of d-T3 with and without aspirin use to prevent colon polyps.

### d-T3 suppresses CRC and adenoma formation in AOM rat.

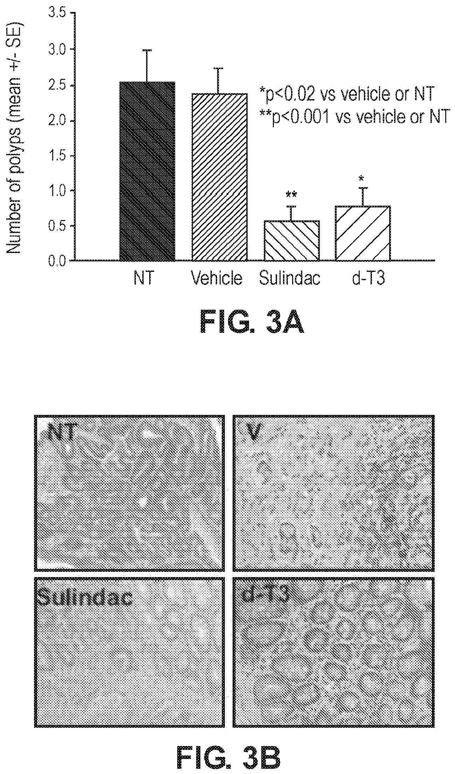

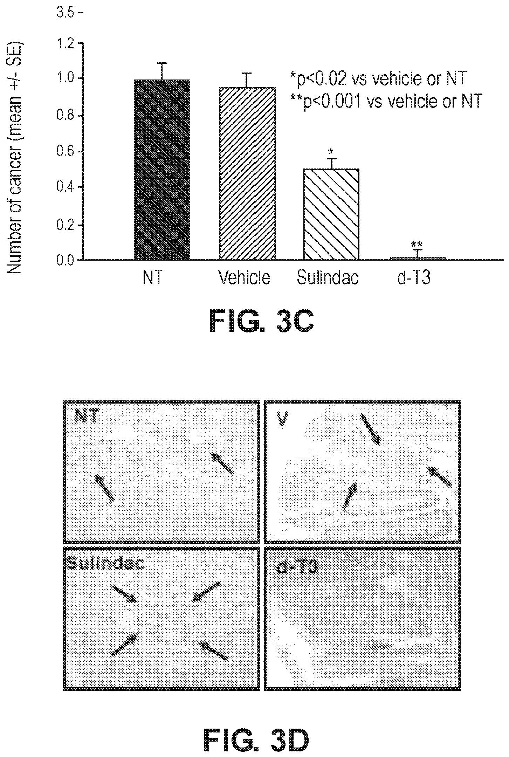

To confirm the CRC prevention activity of d-T3, the activity of d-T3 was investigated and compared this with sulindac, another established NSAID with chemoprevention activity against CRC in the AOM induced rat colon cancer model. As shown in Fig. 3, d-T3 significantly inhibited the formation of intestinal polyps and almost completely blocked cancer formation in this model.

### Bioactive levels of d-T3 can be achieved in humans.

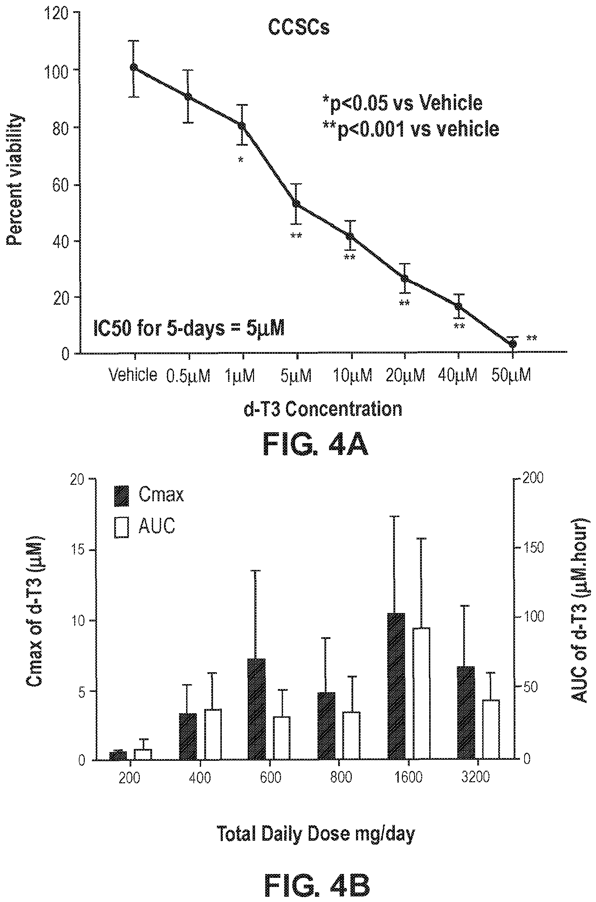

Two phase I dose-escalation studies were performed determining safety, pharmacokinetics, and pharmacodynamics after twice daily oral administration of d-T3 for 14 consecutive days before surgery in patients with resectable pancreatic exocrine neoplasia and in healthy individuals. The results show that d-T3 was well tolerated from the starting dose of 200 mg up to 3200 mg/day without any evidence of toxicity (no dose-limiting toxicities, no drug-related adverse events, and no changes in rate of post-operative complications). The half-life of d-T3 was approximately 4 hours. d-T3 was bioavailable with serum Cmax levels as high as 17 µM and AUC as high as 175 µM hour (Fig. 4B). It should be noted that the data demonstrated that the IC50 for d-T3 in colon cancer stem cells (CCSC) is 5 µM with daily fresh media change as shown in Fig. 4A when cells are exposed to treatment for 5 days. It should be noted that higher IC50 doses of d-T3 (50 µM) were used in some of the *in vitro* experiments simply for the convenience of inducing results with a shorter (24 hour) treatment exposure. Similar d-T3 effects were observed with the short-term high-dose exposure and the long-term low-dose exposure. Bioactive levels (defined as significant selective activation of apoptosis in neoplastic cells compared with normal cells as measured by caspase 3 staining) of d-T3 were observed in the pancreatic neoplasia trial at doses of 400, 600, and 800 mg per day. Based on experience from clinical trials and in animal studies, a dose of 400 mg twice daily was selected for the trial. It should be noted that d-T3 concentration is several times higher in fatty tissues such as the pancreas and colon.

### a) A biorepository and organoids from CRC patients.

The project takes advantage of a unique infrastructure and prospective population-based study termed Total Cancer Care (TCC), TCC is a patient-focused approach to partner with patients with the goal of improving treatments and preventing cancer. The TCC protocol is a research study that enrolls patients prospectively at the onset of their oncology care and follows them through their entire journey of diagnosis, treatment, and surveillance. The patients consent to provide their healthcare information and materials from their tumors and tissues to undergo genetic finger-printing. TCC information and tissues are stored in a unique health and research informatics infrastructure. More importantly, and relevant to this project, the patients agree prospectively to be contacted for participation in clinical trials that can be relevant to improving outcomes of their disease. 132,0 00 cancer patients have enrolled in TCC, with 41,253 tumors collected and 16,279 tumors genetically "finger-printed."

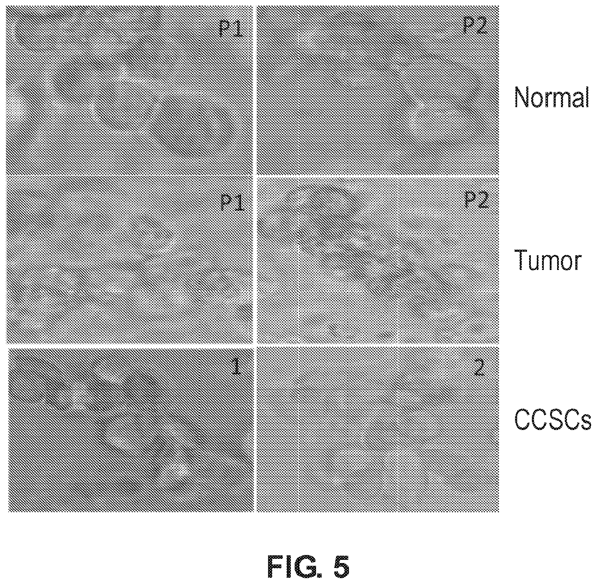

Human intestinal stem cells can be grown "indefinitely" in vitro as 3-D organoids in medium containing the stem cell nitch factors Wnt, Rspondin, EGF, and Noggin while remaining genetically stable. It has been demonstrated that these 3-D organoid cultures derived from healthy and tumor tissue from CRC patients can be used for a high throughput drug screen to identify gene-drug associations that can facilitate personalized therapy. To establish the feasibility of generating organoids from CRC patients, organoids were isolated from normal and cancer tissue from patients undergoing CRC surgery (Fig. 5). Organoids were also established from CCSCs in culture (Fig. 5).

Based on the compelling preclinical and early-phase clinical trial data outlined above, a study can be conducted to investigate whether d-T3 and its combination with aspirin are effective for CRC prevention. This is a single-arm, single-institution, open-label prospective cohort study of the safety and the ability of d-T3 in preventing colon polyps in patients in remission after treatment of stage I-III colon cancer.

Patients in remission after treatment of colon cancer have an increased risk of developing colon cancer through the growth of colon polyps. Reduction of the rate of polyp formation in this patient population is a well-established surrogate intermediate end point biomarker of CRC prevention. A prospective randomized clinical trial has established that the rate of polyp formation in patients with stage I-III colon cancer at 1-year colonoscopy following treatment is 27%. In this study, treatment with low-dose aspirin decreased this rate to 17%. Patients treated with d-T3 are stratified to 1) patients who do not use aspirin and will not consume aspirin and 2) patients who are taking low-dose aspirin for cardiovascular indications, with 75 patients per group. The comparator is all of the other patients (n=75) who are not consuming aspirin, d-T3, or other supplements that may influence polyp formation and thereby is on the observation arm. Based on a retrospective review of the patient population, approximately 25% of these patients are aspirin users. This study design also assesses the safety of d-T3 in aspirin users and nonusers with long-term (6 months) treatment and to access patient tissues for pharmacokinetic and pharmacodynamic studies. To have access to abundant tissue to determine biomarkers and d-T3/metabolites in colon tumors and adjacent colon tissues, a short presurgical (phase 0) treatment (14 days) of 30 patients [15 patients take d-T3 (400 mg twice daily) and 15 take placebo] can be conducted. Finally, advantage can be taken of this prospective cohort study to create a biorepository of well-annotated patient-derived tissues from which PDOs can be generated.

### Example 2: The efficacy and mechanisms of CRC prevention by d-T3 alone and in combination with aspirin in cell and animal models.

### d-T3 augments the ability of aspirin to counteract CCSC traits and targets CCSC factors.

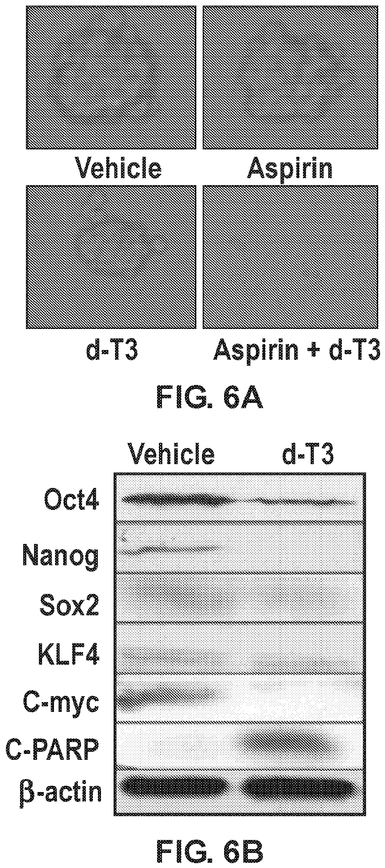

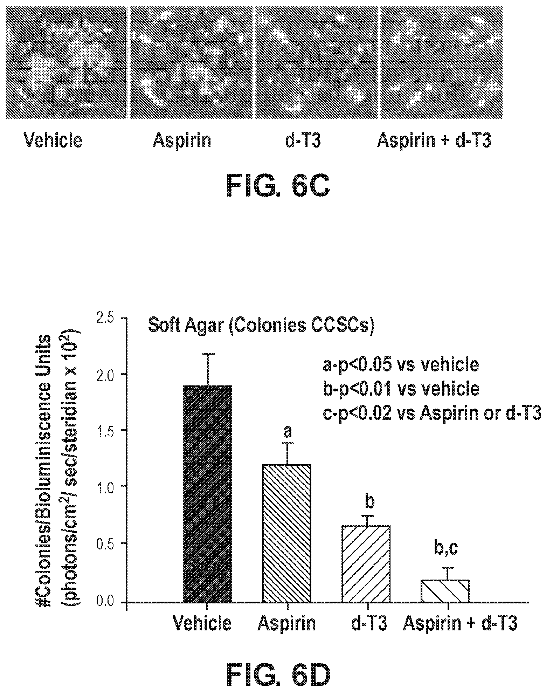

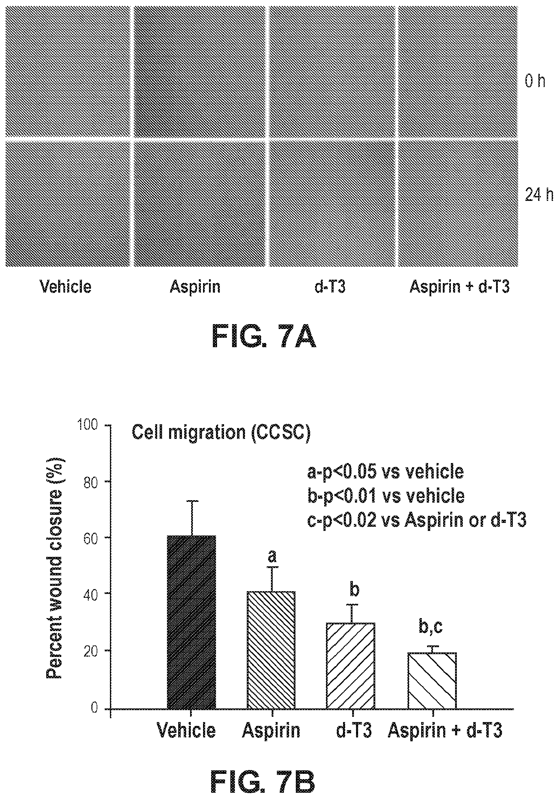

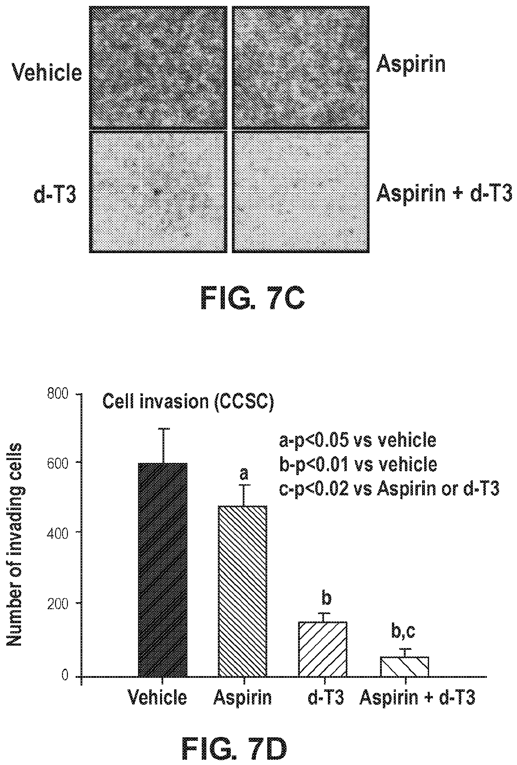

*APC* mutation and β-catenin activation are known to start in colon stem cells at the crypts of the colon mucosa which are transformed to CCSCs. An essential functional effect of the aberrant signaling is disruption of CCSC homeostasis, which leads to overgrowth of the transformed CCSCs, leading to accumulation of additional genetic mutations and progression in colorectal carcinogenesis. Based on the observation of d-T3 and aspirin activity in *Apc*Min/+ mice (see Figs. 1 and 2), further studies were conducted in an *in vitro* and *in vivo* model of human CCSCs. Using human CCSCs purchased from Celprogen (San Pedro, CA), an *in vitro* and *in vivo* orthotopic model of human CCSCs was created by stably transfecting the cells with lentivirus expressing luciferase. These cells were used to conduct investigation of the effect of d-T3 and aspirin treatment on CCSC traits *in vitro.* The combination of d-T3 and aspirin also effectively (synergistically or additively) inhibited CCSC soft agar colony formation (Fig. 6C) and spheroid formation (Fig. 6A), as well as CCSC migration (Fig. 7A) and invasion (Fig. 7C). d-T3 inhibition of CCSC traits is associated with downregulation of Oct4, Nanog, Sox2, KLF4, and c-myc, as well as the upregulation of c-PARP (Fig. 6B). These findings strongly indicate that d-T3 inhibits the CCSC phenotype and strongly augments aspirin inhibition of CCSC traits.

### d-T3 inhibits the growth and metastases of human CCSCs in mice and significantly enhanced aspirin activity.

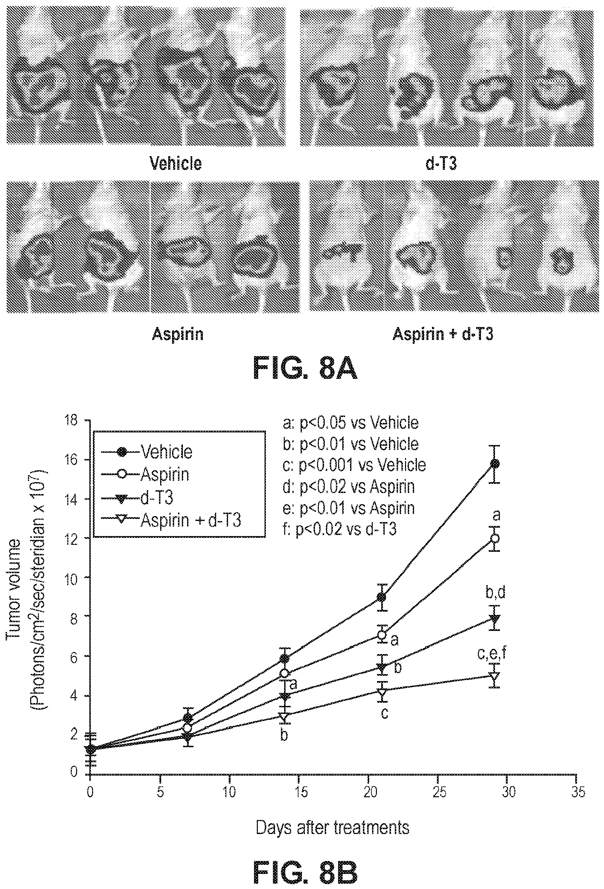

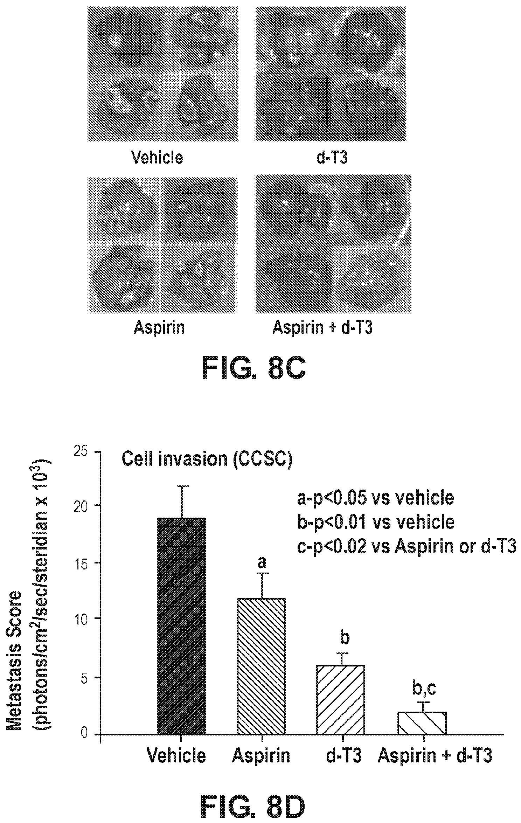

*In vivo* model of human CCSC were developed by implanting the stably transfected luciferase expressing CCSCs in the cecum of NOD-SCID mice. In this model, tumor growth, metastasis, and survival are monitored twice weekly by IP injection of luciferin (150 mg/kg body weight) and imaged using the IVIS 200 Bioluminescence imaging system. As shown in Fig. 8, treatment of these mice with d-T3 alone or aspirin alone inhibited the growth and metastases of human CRC stem cells. However, combination of d-T3 and aspirin virtually eliminated the development of metastases. These findings strongly indicate that d-T3 targets CCSCs and enhances the ability of aspirin to target these cells.

### d-T3 inhibits β-catenin signaling.

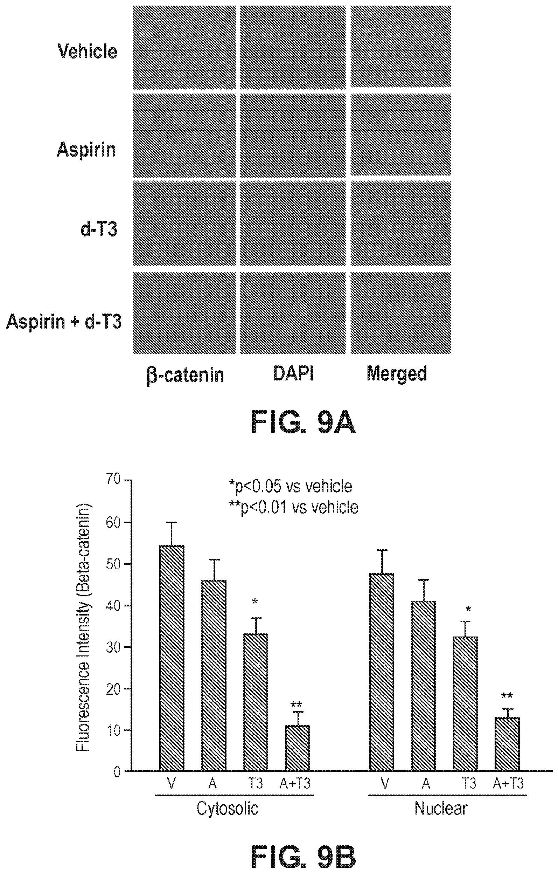

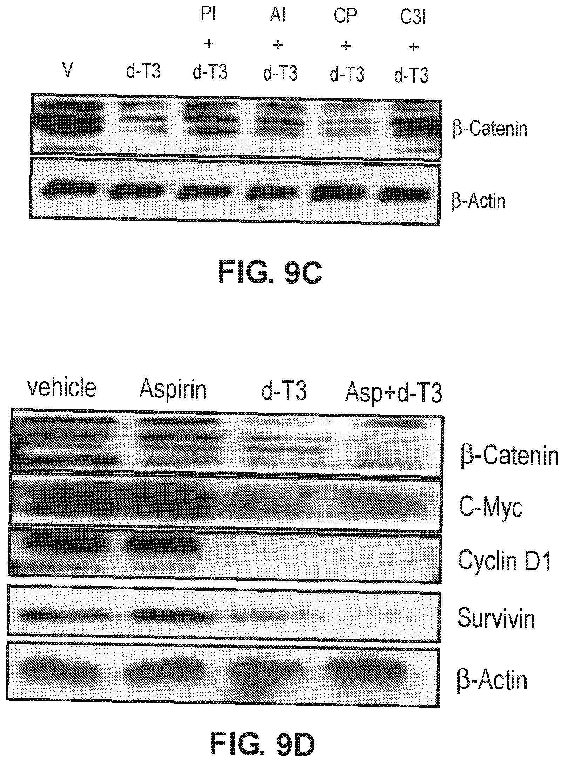

As noted above, stability of the β-catenin complex by inactivation of *APC* leads to stabilization and nuclear translocation of β-catenin and to transcriptional activation of β-catenin target genes such as c-Myc and cyclin D1. As shown in Fig. 9 (A & B), d-T3 significantly decreased the amount of β-catenin that is translocated to the nucleus using immunofluorescence staining of β-catenin. Interestingly, the degradation of β-catenin in the cytoplasm and the inhibition of its translocation to the nucleus were even more profound with d-T3 and aspirin treatment in CCSCs. Furthermore, d-T3 inhibition of CCSCs is associated with the downregulation of β-catenin, c-Myc, cyclin D1, and survivin as measured by Western blot in CCSCs (Fig. 9D). Interestingly, d-T3 induced degradation of β-catenin was inhibited by proteasome inhibitor (MG132, 25 µM), autophagy inhibitor (3-methyladenine, 10 mM), and caspase 3 inhibitor (20 µM) but not by calpain II inhibitor (25 µM) (Fig. 9C). These results indicate that d-T3 activity on CCSCs can be related to its effect on β-catenin stability.

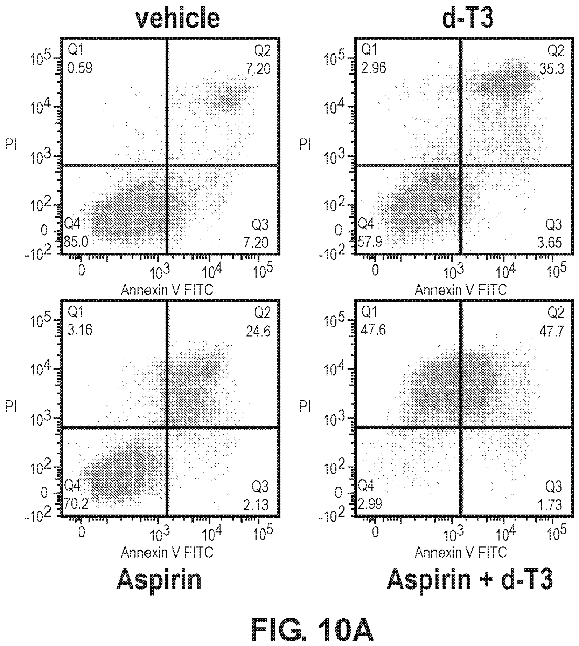

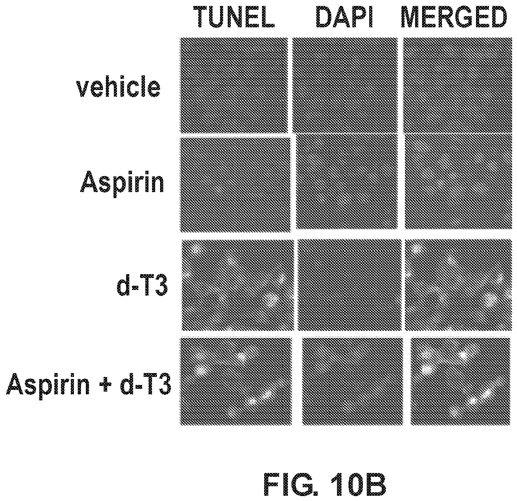

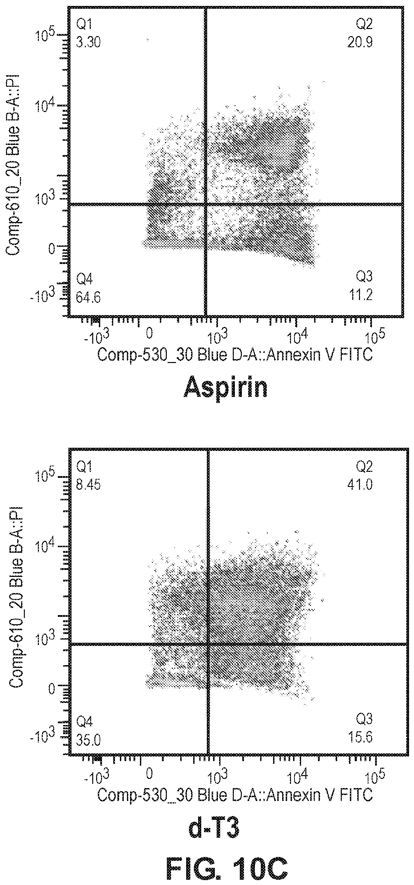

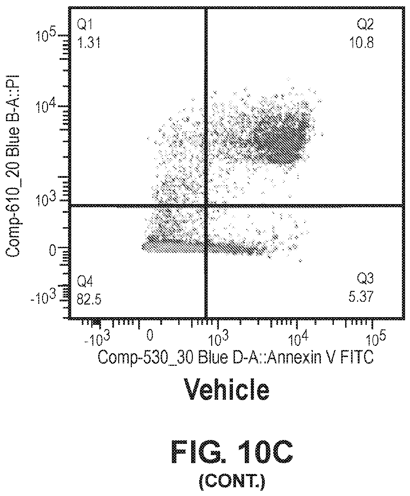

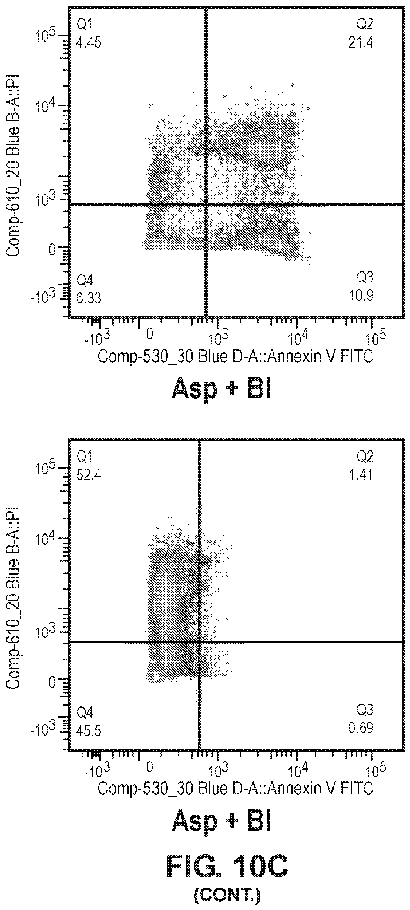

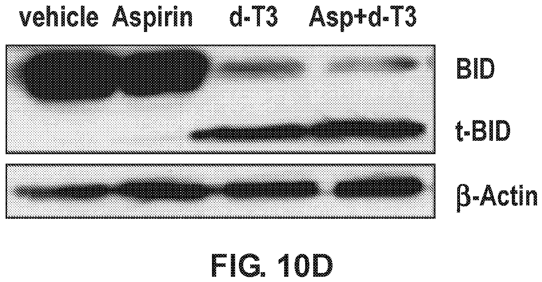

d-T3 can induce selective killing of CCSCs through the BH3 interacting-domain death agonist (BID). In Fig. 10A, it is shown that 24-hour treatment with d-T3 (50 µM) was much more effective than aspirin (5 mM) treatment for 24 hours in inducing apoptosis in CCSCs in an annexin V FITC/PI flow cytometry assay. The combination of aspirin with d-T3 was even more effective (indicative of synergy) in inducing apoptosis in this assay with virtually no live cells remaining (Fig. 10A). It is interesting that the killing of the CCSCs by the combination treatment demonstrated the apoptosis and necrosis mode of cell death. These results were confirmed with TUNEL staining (Fig. 10B). D-T3 significantly induced apoptosis in the human cancer cell lines HCT-116, SW-620, and HT-29 but showed no effect on immortalized normal colon cancer cell line NCM-460. It should be noted that these *in vitro* results are consistent with the *in vivo* observation of the selective induction of apoptosis in APC mutant colon crypt cells in the ApcMin/+ mice but not in the normal intestinal cells of the AOM rat CRC model or NOD/SCID mice. Furthermore, in experiments conducted with Cyp1a humanized mice, colon tumorigenesis induced by 2-amino-1-methyl-6-phenylimidazo(4,5-b)pyridine (PhIP) was effectively induced by 0.025% and 0.05% d-T3 in the diet. The inhibition was associated with the decreased level of COX-2, β-catenin, and protein levels, as well as increased apoptosis. These data strongly implicate selective induction of apoptosis as an important mechanism of d-T3 activity in colon carcinogenesis. Although the proapoptotic activity of d-T3 is a consistent observation of d-T3 anticancer activity in all of the published reports, the mechanism by which d-T3 induces apoptosis is poorly understood. Apoptotic death is regulated by the death receptor (extrinsic) and mitochondrial (intrinsic) pathways. These 2 pathways crosstalk under certain conditions through caspase-8-mediated cleavage of BID, a BH3-only Bcl-2 family member. The activated and truncated BID (tBID) then engages the intrinsic pathway for efficient apoptosis induction. To obtain preliminary information regarding the mechanisms by which d-T3 induces apoptosis in CCSCs, the activation of BID was measured after 24-hour treatment with vehicle, aspirin (5 mM), d-T3 (50 µM), and aspirin combined with d-T3 in CCSCs by Western blotting. As shown in Fig. 10D, d-T3 significantly activated tBID while aspirin did not. Consistent with these results, it is shown in Fig. 10C that treatment of CCSCs with the BID inhibitor BI-6C9 (Santa Cruz Biotechnology, CA) negates the induction of apoptosis by d-T3 but not by aspirin. These results support d-T3 prevention of colon carcinogenesis and enhancement of aspirin colon chemoprevention is at least due in part to induction of apoptosis in CCSCs through activation of BID.

To complement the human studies the efficacy and mechanisms of CRC prevention can be observed in three cell and animal models: *a*) human colon cancer stem cells (CCSC) in culture and in xenograft model. This is a basic experimental system and is suitable to determine whether d-T3 and aspirin interact synergistically or additively to inhibit CCSC growth in culture and in xenografts (including metastasis) and the mechanisms involved. b) CRC patient-derived organoid (PDO) model. The advantage of this model is to determine whether d-T3 and aspirin can be applied to different patients and possible variations among patients due to differences in their oncogenic changes. *c*) ApcMin/+ mice without and with DSS treatment. This provides a well-established animal model for intestinal and colon cancer to illustrate the inhibitor activity of d-T3, aspirin, and their combination. These model systems can provide more detailed mechanistic information and interaction between these two agents and complements the human studies. Based on the results, the efficacy and mechanisms of d-T3 with and without aspirin actions are investigated.

### Example 3: Determine the mode of actions of d-T3 and aspirin in CCSCs in culture, organoids, and xenografts

### CCSC in culture and organoids

CCSC (from Celprogen) can be cultured in the presence of d-T3 and aspirin using established conditions as demonstrated herein. The number of viable cells can be determined by MTT assay. For dose response studies, d-T3 (e.g., 1, 2, 4, 8, 16, and 32 µM) can be added to the daily changed fresh culture media for 3-5 days. The dose response for aspirin can be conducted similarly except with higher doses (1-8 mM).

For analyzing the mode of interaction between d-T3 and aspirin in cell lines, the approach used is one of determining Median Effect and Interaction Index. In this Media Effect Plot analysis, Log (E/(1-E)), where E is the fraction of cells that survived, is plotted against Log(Dose). Based on this Media Effect Plot, d1, d2, Dx,1, and Dx,2 are obtained (d1 and d2 are the doses of d-T3 and aspirin in the combination treatment that produce certain E; and Dx,1 and Dx,2 are the doses of d-T3 and aspirin in single treatments that produce the same E). Interaction index (II) is calculated for different E values using II = d1/Dx,1 + d2/Dx,2. In final result analysis, II = 1, <1 or >1 indicates additivity, synergy, or antagonism of the combination, respectively. As an example, the different concentrations of d-T3 can be 1, 2, 4, 8, 16, and 32 µM. For the combination, the d-T3 to aspirin ratio can be 1:100 (or an experimentally determined value). The concentrations are be adjusted based on data.

In studies with CCSC organoids, previously established conditions are used and the number of colonies formed can be analyzed. The dose response and interactions between d-T3 and aspirin can be conducted as described above. The information obtained from the above studies can help to simplify the design of future studies.

### CCSC xenografts.

The CCSC xenograft model can be established and monitored as described above. Luciferase expressing CCSCs (1 × 106 cells/mouse) can be suspended in Matrigel and then injected into the cecum of NOD-SCID mice. Mice with palpable tumors can be euthanized (date of death recorded), and the tumors can be harvested, weighed, measured, and assessed using morphologic parameters. At the end of 3 weeks of treatment, 5 of the 15 mice from each group can be injected with BRDU and 2 hours later tumor tissue in the colon and spleen as well as colon, liver, and lung tissue can be harvested. Tumors can be analyzed for proliferation, apoptosis, stem cell markers, and other d-T3 target genes. At the end of the treatment, blood and serum from all animals as well as tumor tissue, pancreas, liver, and lung tissue can be harvested and frozen for biochemical and immunohistochemical assays.

For determining interaction between two agents in animal studies, the design has the following groups based on the AIN93M diet (per kg) containing: G1, no d-T3 (control), G2 - 0.5 g d-T3, G3 - 1g d-T3, G4 - 2g d-T3, G5 - 0.05 g aspirin, G6 - 0.1g aspirin, G7 - 0.2g aspirin, G8 - 0.5g d-T3 + 0.05g aspirin, and G9 - 1g d-T3 + 0.1g aspirin. The design assesses the dose responses of the two agents and their interactions. If the inhibitory effect of G8 is larger than that of G3 and G6, for example, it indicates synergistic effects.

### Analysis of biomarkers from mechanistic information.

It is tested herein that d-T3 inhibits CRC formation by inhibiting aberrant Wnt signaling and enhancing apoptosis. The biomarkers to be analyzed in the xenograft tumors can mimic the analysis in humans as described in section Id and can have the advantages of having reproducible high quality samples for analysis. In the studies with xenograft the d-T3, other vitamin E forms, and their metabolites in the blood, urine, nontumorous colon, and fecal tissues can be analyzed. Correlation analysis between colonic levels with those in blood, urine, or fecal samples can be conducted as described. D-T3 metabolite levels can also be correlated with inhibition of CCSCs.

### Example 4: Identify the mechanisms by which induction of apoptosis by d-T3 enhances aspirin activity in the inhibition of colon carcinogenesis

d-T3 treatment significantly induces apoptosis in CCSCs and that inhibition of BID abrogated the ability of d-T3 to induce apoptosis in CCSCs. A comprehensive experimental approach can be used to determine whether or not BID mediates selective killing of APC deficient cells in intestinal tumor suppression by d-T3 and aspirin.

To determine whether BID is required for the chemopreventive effects of d-T3 with or without aspirin, Igr5-EGFP expressing *ApcMin*/+ mice can be crossed with *BID* knockout mice and generate ageand sex-matched cohorts of *ApcMin*/*+* mice with different BID genotypes. These mice can be obtained from the Jackson Laboratory. The mice can be treated with vehicle, d-T3, aspirin, and combination of d-T3 with aspirin. The doses of these compounds can be determined from the experiments described above. Small intestinal and colon polyps can be assessed and compared between treatment groups as shown in the data. Also the effects of prolonged treatment can be compared on the survival of *BID*+/+ and *BID*-/*- ApcMin*/+ mice. Results from these experiments demonstrate whether BID plays an essential role in d-T3-mediated chemoprevention in *ApcMin*/+ mice. To determine whether BID is required for d-T3-induced killing of intestinal stem cells in *ApcMin*/+ mice, the killing effect of d-T3 with or without aspirin can be measured by TUNEL/EGFP double-positive staining in the small intestine and colon of *BID-*/*-ApcMin*/+ mice relative to *BID*+/+*ApcMin*/+ mice.

Results from these experiments indicate whether or not BID mediates the chemopreventive effects of d-T3 and aspirin through selective killing of *APC*-deficient intestinal stem cells. To further delineate the functional role of BID in d-T3-mediated tumor suppression, HCT-116 colon cancer cells can be analyzed, which contain a β-catenin-activating mutation and appear to recapitulate the anticancer and apoptotic effects of d-T3 in mice. An inducible *BID* knockout (*BID* KO) HCT-116 cell line can be generated by using lentiviral expressing *BID* shRNA. Parenteral, induced, and not induced *BID* KO cells can be compared for their responses to vehicle, d-T3, aspirin, and d-T3 with aspirin treatment. Apoptosis can be assessed, as well as various markers of the mechanisms of both intrinsic and extrinsic apoptosis. This in vitro system can be complemented by other cell lines, including CCSCs, APC-mutant DLD1 and HT29 cells, and APC-WT RKO cells. Experimental controls can include other agents such as tocopherols, tocotrienols, other NSAIDs, and TRAIL. Results from these experiments demonstrate a prominent and specific role of BID in mediating the anticancer and apoptotic effects of d-T3. To confirm whether activation of BID by d-T3 involves the most common activator of BID, caspase 8, whether chemical inhibition all knockdown of caspase 8 blocks d-T3-induced apoptosis can be determined. Finally, to validate the relevance of the mechanistic studies in humans, apoptosis (TUNEL/caspase 3), active caspase 8, and tBID can be compared in the advanced adenomas of the 3 patient cohorts.

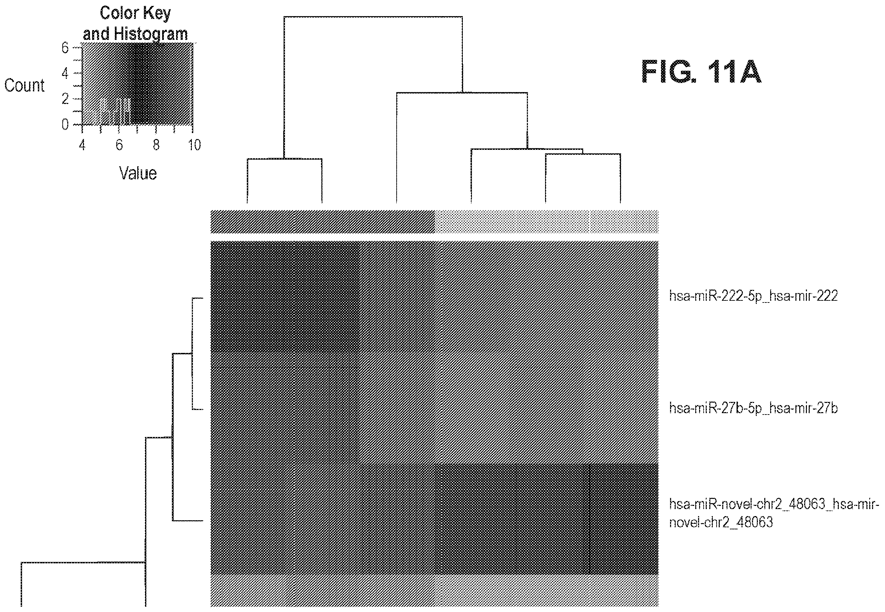

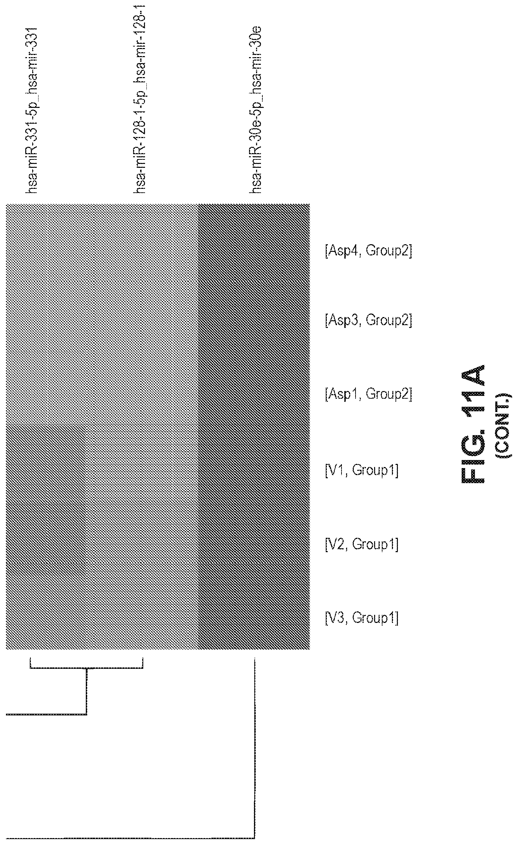

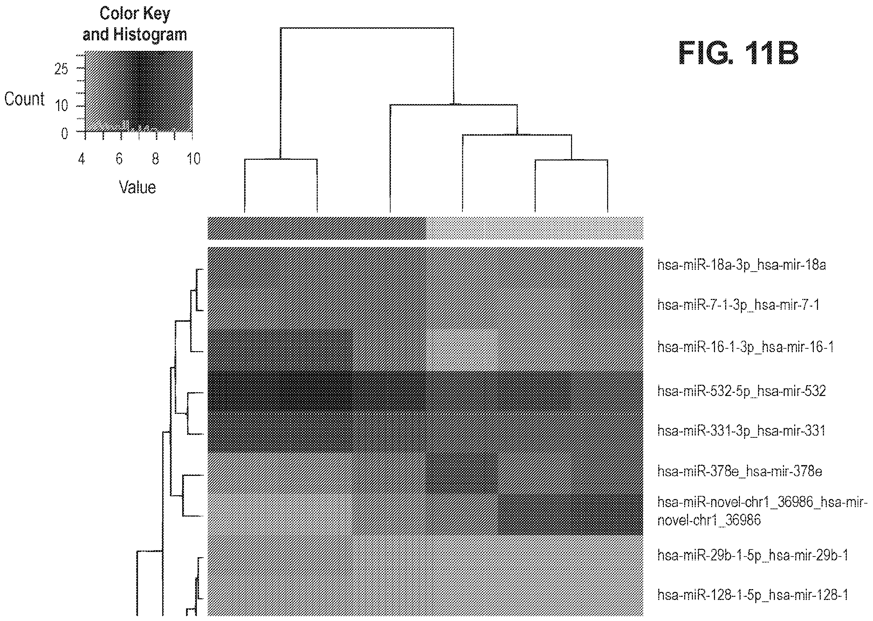

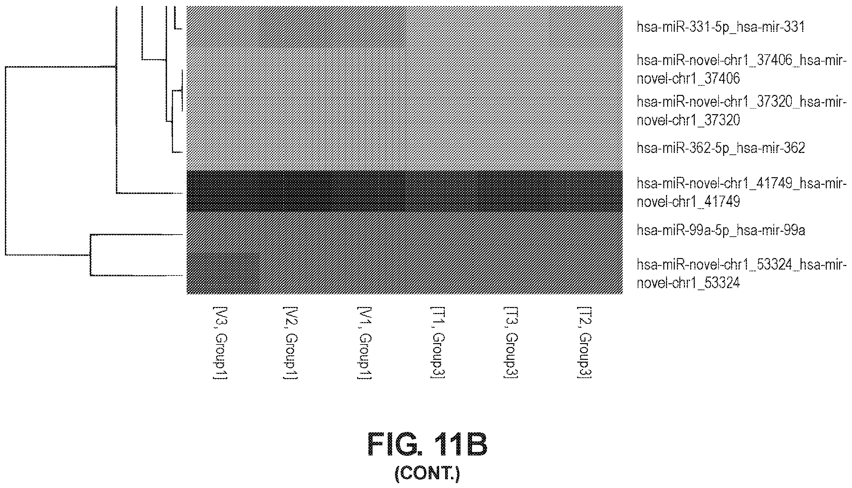

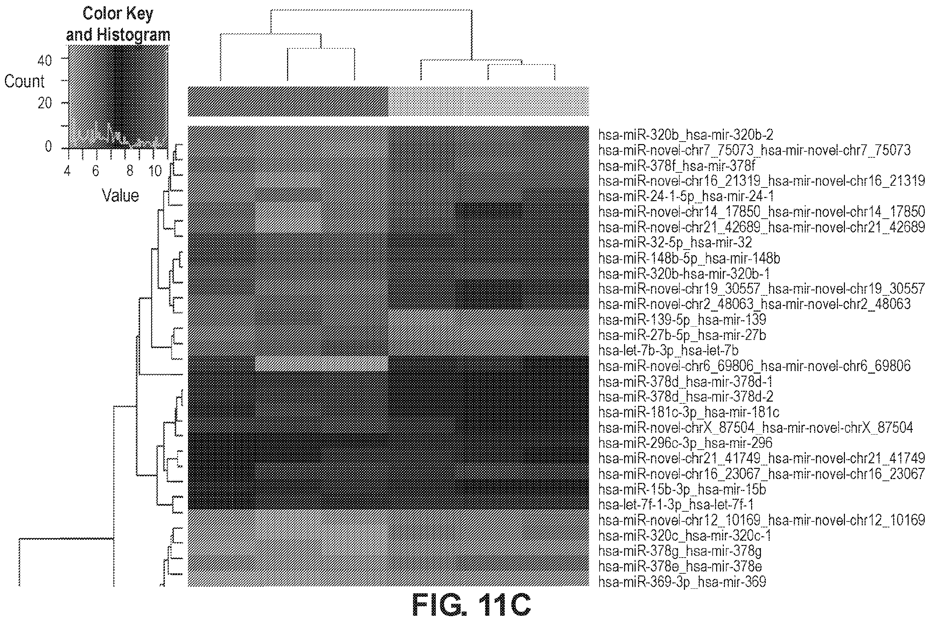

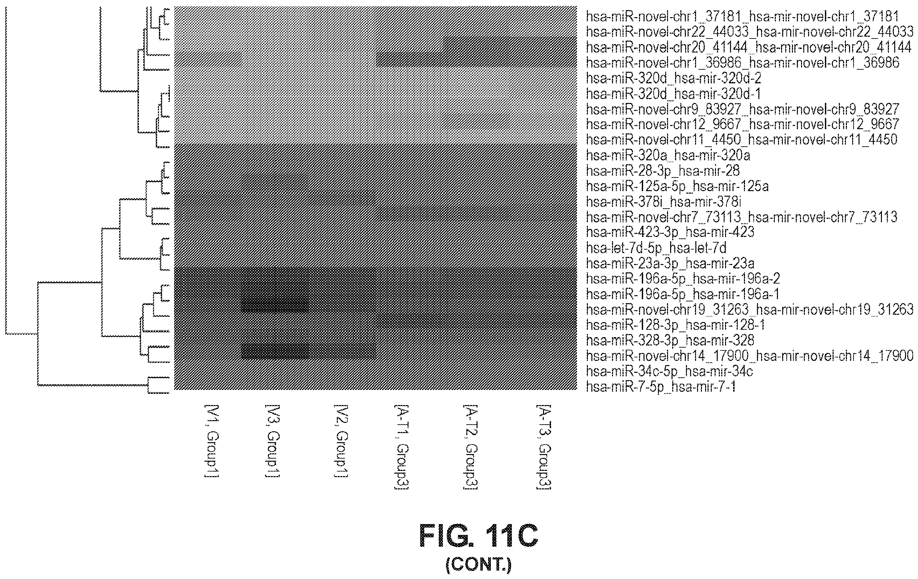

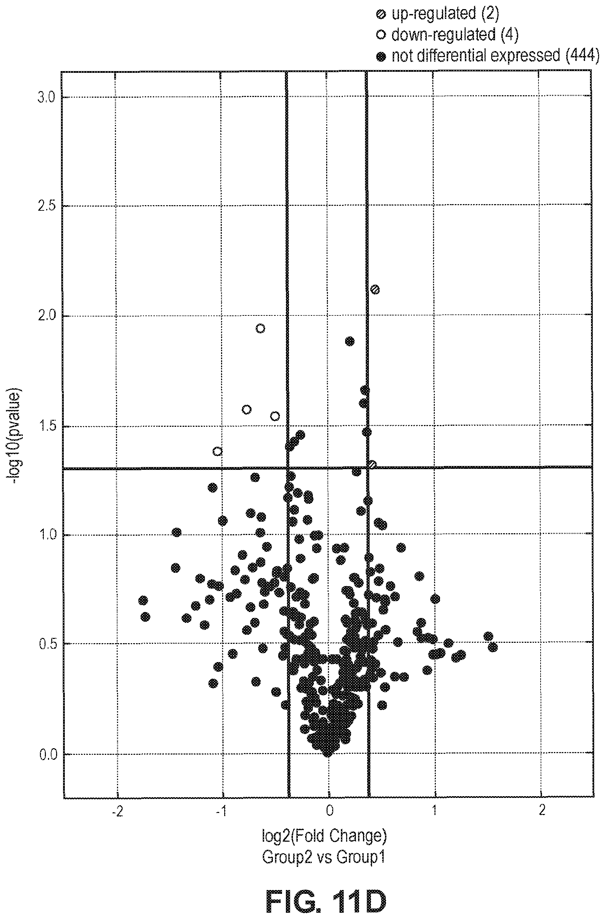

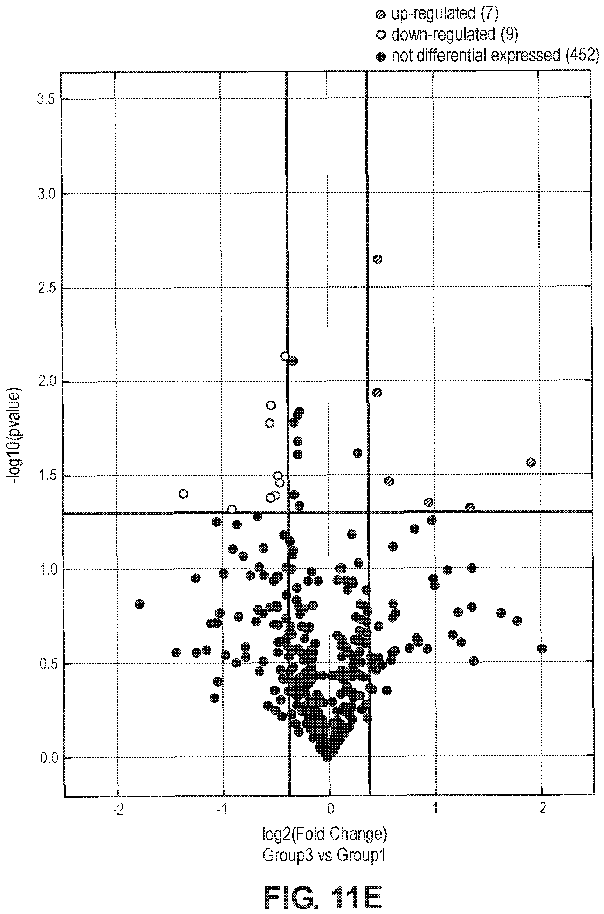

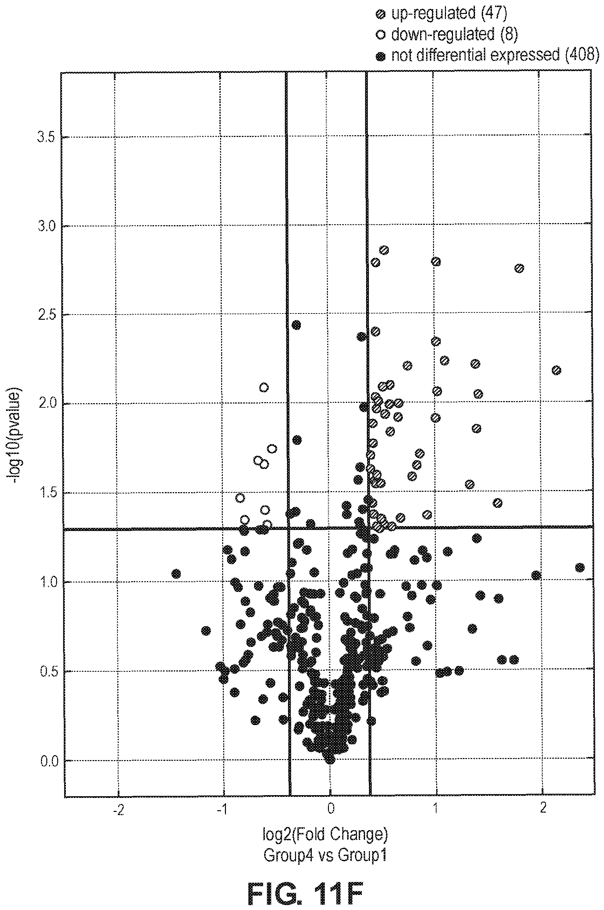

Given the results, and without intending to be bound by any theory, it appears that the induction of apoptosis can be one mechanism by which d-T3 prevents CRC and augments CRC prevention activity. BID knockout can diminish this effect. The biorepository as well as materials generated from studies can be used to explore other mechanisms of d-T3 action such as antiinflammatory, mediation of the immune system, anti-angiogenesis, and alterations in metabolism. To this end, miRNA profiles were conducted that are altered with d-T3, aspirin, and d-T3 with aspirin treatment in CCSCs. To discover early miRNAs that are likely to influence activity, these cells were deliberately treated with doses of these agents at a duration when there were no phenotypic changes of apoptosis. As shown in Fig. 11, 24-hour treatment with aspirin (5 mM) resulted in up-regulation of 2 miRNAs and down-regulation of 4 miRNAs compared with vehicle. In contrast, 24-hour treatment with d-T3 (10 µM) resulted in significant up-regulation of 7 miRNAs and down-regulation of 9 miRNAs compared with vehicle. Interestingly, 24-hour treatment with the combination of aspirin (5 mM) and d-T3 (10 µM) resulted in significant up-regulation of 47 miRNAs and downregulation of 8 miRNAs when compared with vehicle. These exciting results clearly demonstrate that d-T3 combined with aspirin triggers unique transcriptional programs.

### Example 5: Treatment of Cancers Expressing Human Cellular Apoptosis Susceptibility (hCAS) protein with δ-Tocotrienol

It was found that hCAS is over-expressed in human pancreatic preneoplastic and cancer cells but not in normal tissues from human pancreatic biopsies (FIGURE 12). These finding that hCAS is already present in pre-cancerous tissues, cancer stem cells and cells with dysplastic features of varying degree, when combined with the other findings in Example 5, establishes a sound treatment rationale for patients at risk of metastasis or cancer recurrence.

### Inhibition/Reduction of hCAS induces cancer cell death

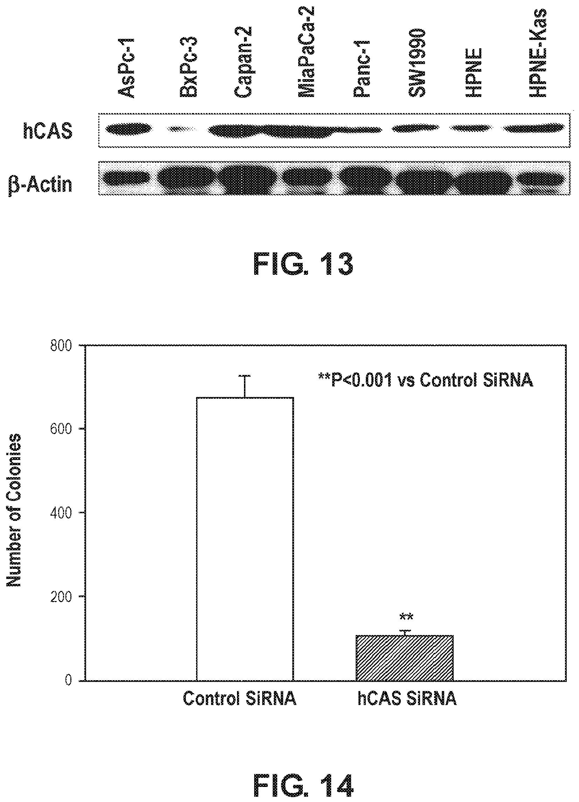

The hCAS protein has previously been identified as having high expression levels in cancer cells. hCAS plays a pivotal role in nuclear/cytoplasmic transport and its overexpression is associated with metastasis and poor patient outcomes (see more below). hCAS is expressed in several pancreatic cancer cell lines (FIGURE 13), and inhibition of its expression with hCAS SiRNA significantly reduces MiaPaCa-2 cell growth by inhibition of soft agar colony formation (FIGURE 14).

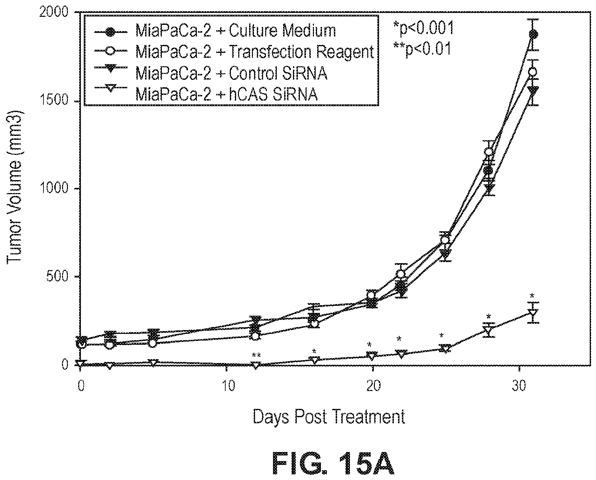

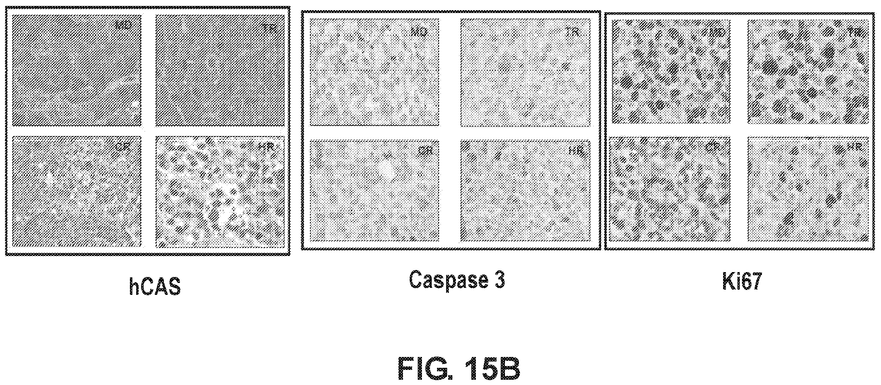

In vivo, reduction of hCAS levels with hCAS SiRNA also significantly inhibited MiaPaCa-2 xenograft tumor volume growth in nude SCID mice (FIGURE 15A), identifying hCAS as a viable drug target. Importantly, tumor biopsies from these mice show that depletion of hCAS resulted in increased apoptosis (caspase 3) and decreased proliferation (Ki67) as can be seen in FIGURE 15B.

### δ-Tocotrienol (δ-T3) Reduces hCAS-levels and Induces Cancer Cell Death

Compounds that kill cancer cells by inhibiting or reducing hCAS expression levels have not been described before.

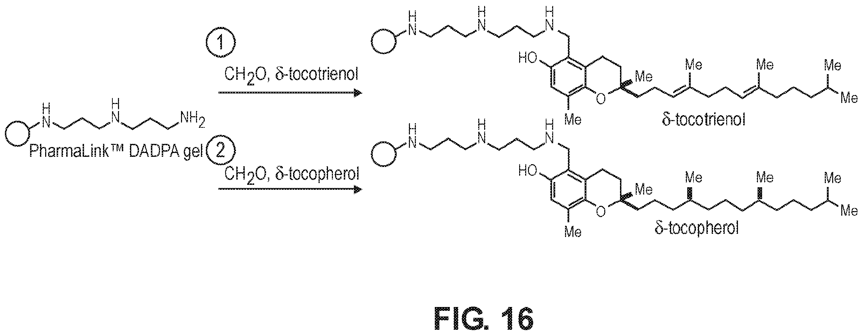

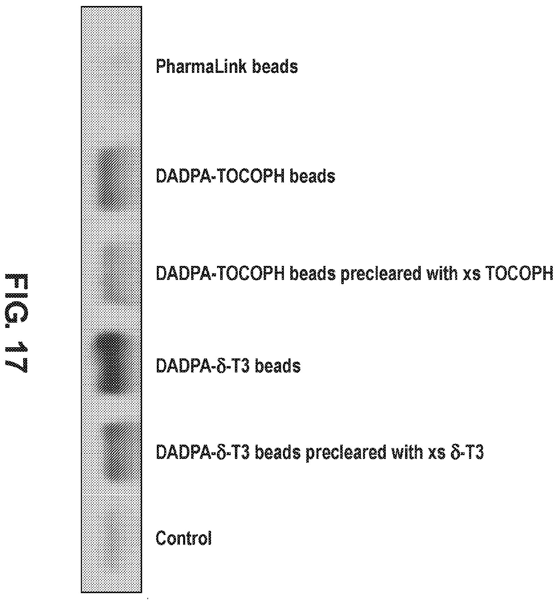

Affinity gels with δ-T3 were prepared by coupling the δ-Tocotrienol (δ-T3) R1 position and the δ-Tocopherol (TOCOPH) R1 position (FIGURE 16) with the free amino group of diaminodipropylamine (DADPA) functionalized cross-linked Agarose beads (Pharmalink^{™}hkit, (Pierce)). These affinity gels were incubated with lysates from MiaPaCa-2 cell lysates, and the captured proteins were identified by mass spectrometry (FIGURE 17).

This experiment used a synthetic biotin-δ-tocotrienol conjugate (shown below to inhibit growth activity of cultured MiaPaca-2 human pancreatic cancer cells) to confirm affinity-isolation of hCAS. To confirm that δ-T3 binds to hCAS in intact cells, biotinylated δ-T3 (B-δ-T3) was prepared by chemically coupling the -OH group of δ-T3 to the -COOH of biotin using a PEG-iodoactamide linker (FIGURE 18). TOCOPH, the inactive isoform of δ-T3, was biotinylated similarly to obtain B-TOCOPH and used as a control. Affinity-isolation of hCAS was inhibited in the presence of free δ-tocotrienol but not with free δ-tocopherol.

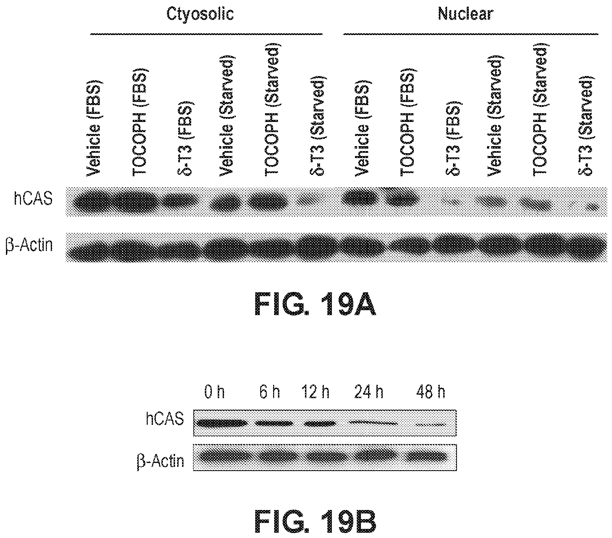

MiaPaCa-2 human pancreatic cancer cells were treated for 24 hours with biotin alone (B), B-δ-T3 or B-TOCOPH. The cells were then lysed, and the lysates incubated with streptavidin beads, and the captured proteins processed for Western blotting. FIGURE 18 shows that streptavidin beads bound hCAS from lysates of MiaPaCa-2 cells treated with B-δ-T3, but not B-TOCOPH or biotin alone, demonstrating that δ-T3, but not TOCOPH, binds hCAS in intact pancreatic cancer cells. To determine whether the interaction of δ-T3 with hCAS has functional consequences, MiaPaCa-2 cancer cells were treated with δ-T3, and it was determined whether δ-T3 affects the cellular levels of hCAS in both Cytostolic and Nuclear compartments (FIGURE 19A). These results were confirmed with immuno-fluorescence microscopy studies where B-δ-T3, but not B-TOCOPH, co-localized with hCAS in both the nucleus and cytosol of treated cancer cells (data not shown), confirming the above Western results of FIGURE 19A.

FIGURE 19B shows that treatment of MiaPaCa-2 cells with δ-T3 decreases the hCAS levels starting at 6 hours with a maximum effect at 24-48 hours.

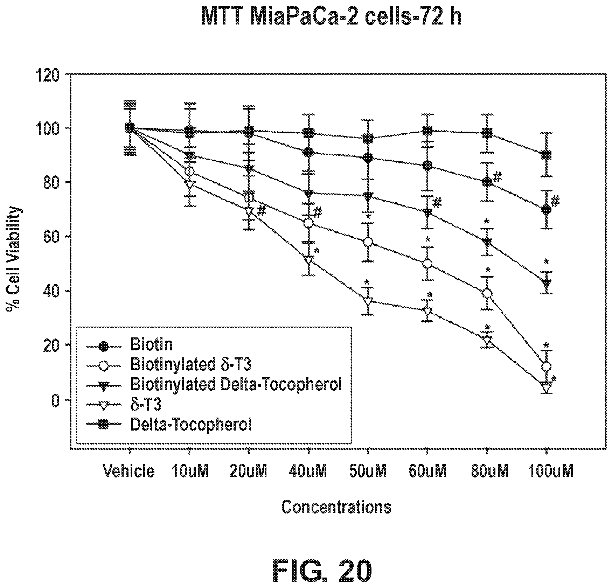

When verifying the relative dose-dependent effects on MiaPaCa-2 cell viability, increasing TOCOPH concentrations had the least effect on cell viability (even less than biotin), while free δ-T3 and B-δ-T3 were most potent in reducing cell viability, killing almost all cells at 100 µM concentration (FIGURE 20).

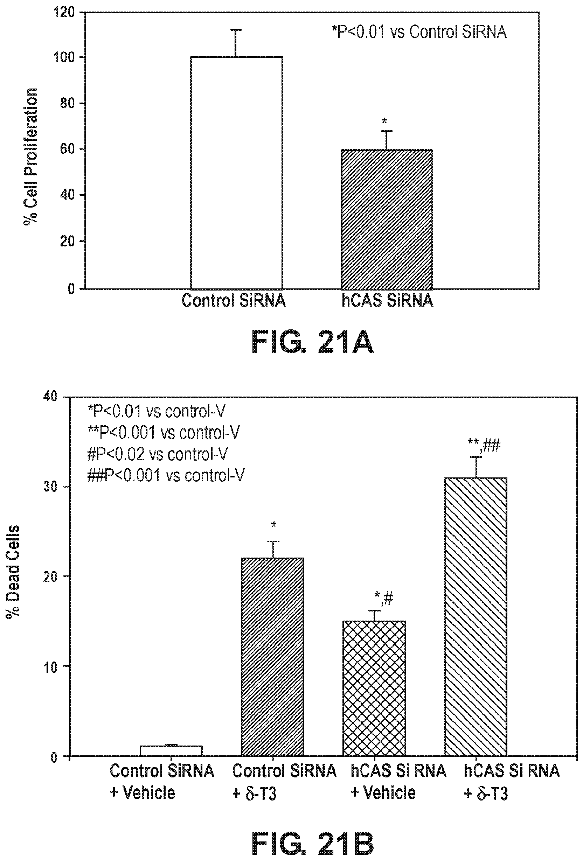

Next, MiaPaca-2 cells were transfected with Non-Targeting (Control) or hCAS siRNA (Cat# 2402879, Qiagene), showing that hCAS inhibition significantly knocks down cell proliferation (FIGURE 21A). As compared to control SiRNA, the combination of control SiRNA + δ-T3 induces cell death in MiaPaCa-2 cells. The latter occurs at even a higher rate than by knocking down hCAS with hCAS siRNA, illustrating the significant potency of δ-T3. The combination of hCAS SiRNA + δ-T3 was clearly most potent inducing the highest level of cell death (FIGURE 21B). The ability of δ-T3 to add to the already potent effect of hCAS SiRNA illustrates the potency and utility of δ-T3 therapy.

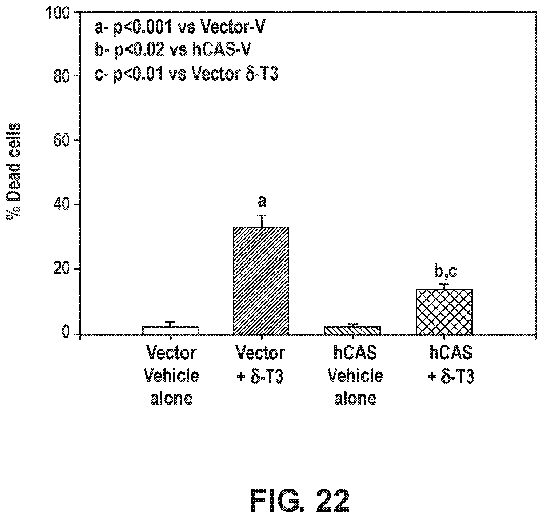

To determine whether hCAS over-expression can rescue from δ-T3 effects, MiaPaCa-2 cells stably expressing hCAS were generated by transfecting these cells with the hCAS gene-containing plasmid pCMV6-AC-GFP (Cat# RG211478, Qiagene) and selecting the cells stably expressing hCAS under G418 pressure. MiaPaCa-2 cells expressing the same plasmid without the hCAS gene were also generated as empty vector control. FIGURE 22 shows that over-expression of hCAS significantly (comparison "c": p<0.01) rescued MiaPaCa-2 cells only partially from δ-T3-induced tumor cell death, and that the δ-T3 treatment still induces significant cancer cell death versus the hCAS vehicle (FIGURE 22; comparison "b": p<0.02). The inability of over-expression to entirely overcome the effect of δ-T3 treatment further illustrates the robustness of a δ-T3 therapeutic approach.

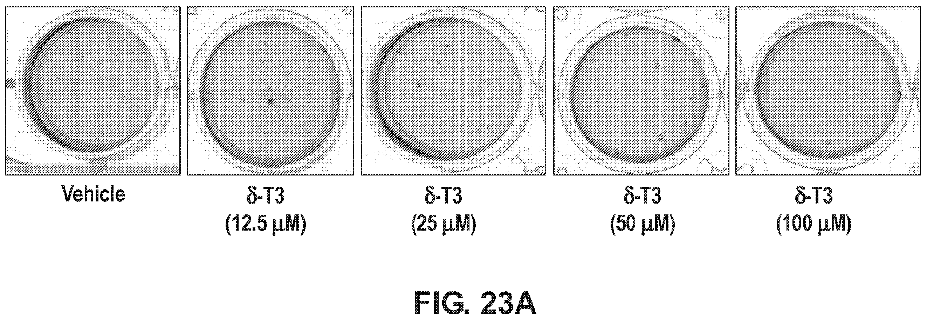

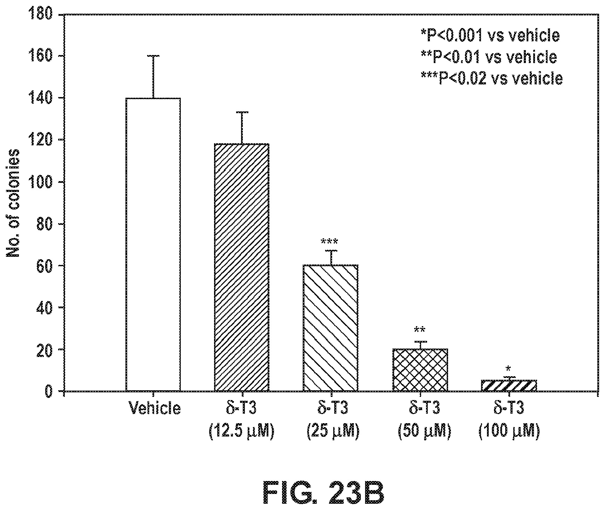

A δ-T3 dose response evaluation conducted in HPNE cells, another pancreatic cancer cell line that expresses hCAS (FIGURE 23), demonstrates a similarly robust dose-response at concentrations similar to those observed with MiaPaCa-2 cells, thus, confirming the ability of δ-T3 inhibit the growth of another hCAS overexpressing cancer cell line.

The experiments in this Example 5 demonstrate that hCAS is a protein that binds to δ-T3 in vitro and in vivo. δ-T3 treatment of pancreatic cancer cells in vitro and in vivo depletes or reduces hCAS, which is involved in the ability of pancreatic cancer and precancerous cells to grow and survive. Thus, the ability of δ-T3 to inhibit malignant transformation and cell growth in an early single cell (cancer stem cell) stage appears to be due at least, in part, to its ability to decrease hCAS levels.

In addition, identifying cancer cells that overexpress hCAS protein, can be used to predict a patient's likelihood to experience metastasis or cancer recurrence and/or to predict whether the cancer will respond to δ-T3 therapy. Screening patients for hCAS overexpression prior to treatment should increase the success rate of δ-T3 therapy.

In view of the above, it will be appreciated that the invention also encompasses the following items:
1. A method of inhibiting metastasis of a cancer, inhibiting a cancer recurrence, or post-anti-cancer treatment maintenance in a subject following cancer therapy, the method comprising administering to the subject a composition comprising a therapeutically effective amount of a compound that reduces hCAS levels in cancerous or pre-cancerous tissues, wherein prior to the administering step, the subject was identified as having increased levels of hCAS expression relative to a control.
2. A method of determining if a subject is at risk for developing cancer metastasis or recurrence, the method comprising assaying a biological sample from the subject to determine a level of hCAS expression in the sample, wherein an increased expression level of hCAS as compared to a control indicates that the subject has an increased risk to develop cancer metastasis or recurrence.
3. The method of item 2, wherein the biological sample comprises blood, serum, plasma, urine or tissue.
4. The method of item 3, wherein the tissue is a primary tumor tissue or a biopsy tissue.
5. The method of any one of items 2-4, further comprising treating the subject for cancer metastasis or recurrence.
6. The method of item 5, wherein the treating step comprises administering a therapeutically effective amount of a compound that reduces hCAS levels in cancerous or pre-cancerous tissues.
7. The method of item 1 or 6, wherein the compound comprises a nucleic acid inhibitor molecule that reduces hCAS mRNA expression levels.
8. The method of item 1 or 6, wherein the compound comprises δ-tocotrienol (d-T3).
9. The method of any one of items 1-8, wherein the cancer is lung cancer, ovarian cancer, breast cancer, rectal cancer, sarcoma, liver cancer, pancreatic or colon cancer.
10. The method of item 9, wherein the cancer is pancreatic cancer.
11. The method of item 9, wherein the cancer is colon cancer.
12. A method of inhibiting metastasis of a cancer, inhibiting a cancer recurrence, or post-anti-cancer treatment maintenance in a subject following surgical removal or anti-cancer treatment of a cancer, the method comprising administering to the subject a composition comprising a therapeutically effective amount of δ-tocotrienol (d-T3).
13. A method of preventing a cancer in a subject at risk thereof comprising administering to the subject a composition comprising a therapeutically effective amount of δ-tocotrienol (d-T3).
14. The method of items 12 or 13, wherein the cancer is lung cancer, ovarian cancer, breast cancer, rectal cancer, sarcoma, liver cancer, pancreatic, or colon cancer.
15. The method of any of items 12-14, further comprising administering to the subject an NSAID or COX-2 inhibitor.
16. A method of treating a cancer in a subject, comprising administering to the subject a composition comprising a therapeutically effective amount of δ-tocotrienol (d-T3), wherein the cancer is lung cancer, ovarian cancer, breast cancer, rectal cancer, sarcoma, liver cancer, pancreatic or colon cancer.
17. The method of any of items 8 or 12-16, wherein the daily dose of d-T3 is between about 400mg and 1600mg.
18. The method of any of items 8 or 12-16, wherein the d-T3 composition is administered twice daily.
19. The method of any of items 8 or 12-16, wherein the composition comprises 400mg of d-T3.
20. The method of any of items 8 or 12-16, wherein the composition comprises 800mg of d-T3.
21. The method of any of items 8 or 12-16, wherein the d-T3 composition is administered for 6 months.
22. The method of any of items 8 or 12-16, wherein the d-T3 composition is administered orally.
23. The method of item 16, further comprising administering to the subject an NSAID or a COX-2 inhibitor.
24. The method of any one of items 12-23, further comprising administering to the subject a therapeutically effective amount of a compound that reduces hCAS levels in cancerous or pre-cancerous tissues.
25. The method of item 24, wherein the compound is a nucleic acid inhibitor molecule that reduces hCAS mRNA expression levels.
26. The method of any one of items 12-25, wherein the cancer is pancreatic cancer.
27. The method of item 15 or 23, wherein the NSAID is aspirin.

## Claims

1. A composition comprising a therapeutically effective amount of δ-tocotrienol (d-T3) for use in preventing a cancer in a subject at risk thereof.

2. A composition comprising a therapeutically effective amount of δ-tocotrienol (d-T3) for use in an anti-cancer treatment maintenance method in a subject following surgical removal or anti-cancer treatment of a cancer.

3. The composition for the use of claims 1 or 2; wherein the subject was identified prior to treatment as having increased levels of hCAS expression relative to a control.

4. The composition for the use of any of claims 1-3, wherein the cancer is lung cancer, ovarian cancer, breast cancer, rectal cancer, sarcoma, liver cancer, pancreatic cancer, or colon cancer.

5. The composition for the use of any of claims 1-4, wherein the daily dose of d-T3 is between about 400 mg and 1600 mg.

6. The composition for the use of any of claims 5, wherein the composition comprises 400 mg or 800 mg of d-T3.

7. The composition for the use of any of claims 1-6, wherein the d-T3 composition is administered twice daily.

8. The composition for the use of any of claims 1-7, wherein the d-T3 composition is administered for 6 months.

9. The composition for the use of any of claims 1-8, wherein the d-T3 composition is administered orally.

10. The composition for the use of any of claims 1-9, further comprising administering to the subject a therapeutically effective amount of a compound that reduces hCAS levels in pre-cancerous tissues.

11. The composition for the use of claim 10, wherein the compound is a nucleic acid inhibitor molecule that reduces hCAS mRNA expression levels.

12. The composition for the use of any of claims 1-11, further comprising administering to the subject an NSAID or COX-2 inhibitor.

13. The composition for the use of claim 12, wherein the NSAID is aspirin.
